# EUROPÄISCHE PATENTANMELDUNG

(11) **EP 2 810 956 A1**
(43) Veröffentlichungstag der Anmeldung: **10.12.2014**
(21) Anmeldenummer: 13170317.5
(22) Anmeldetag: 03.06.2013
(51) Int. Cl.: C08C 19/22, C08L 9/02

(54) **Über Bisdihydropyrazol-Gruppen gekuppelte Nitrilkautschuke, deren Herstellung und Verwendung**

(71) Anmelder: LANXESS Deutschland GmbH, 50569 Köln (DE); Karlsruher Institut für Technologie, 76128 Karlsruhe (DE)
(72) Erfinder: Brandau, Sven, Dr., 67000 Strasbourg (FR); Kaiser, Andreas, Dr., 67000 Strasbourg (FR); Westeppe, Uwe, Dr., 67610 La Wantzenau (FR); Barner-Kowollik, Christopher, Prof. Dr., 76297 Stutensee-Blankenloch (DE); Dürr, Christoph, 69123 Heidelberg (DE); Lederhose, Paul, 77694 Kehl (DE)

(57) **Zusammenfassung**

Die Herstellung eines über Bisdihydropyrazol-Gruppen gekuppelten Nitrilkautschuks erfolgt durch Umsetzung eines Nitrilkautschuks, der auf konjugierten Dienen, α,β-ungesättigten Nitrilen und gegebenenfalls weiteren copolymerisierbaren Monomeren als Monomeren basiert, hydriert sein kann und kovalent gebundene Tetrazolgruppen aufweist, mit einer bifunktionalen En-Verbindung, in der die En-Gruppen mit den Tetrazolgruppen jeweils zu Dihydropyrazol-Gruppen umgesetzt werden können.

## Beschreibung

Die vorliegende Erfindung betrifft Verfahren zur Herstellung von über Bisdihydropyrazol-Gruppen gekuppelten Nitrilkautschuken, die hydriert sein können, nach dem Verfahren erhältliche Kautschuke, über Tetrazol- und En-Gruppen unter UV-Einstrahlung gekuppelte Nitrilkautschuke, deren Verwendung, diese enthaltende vulkanisierbare Mischungen und Vulkanisate sowie Verfahren zur Herstellung der Vulkanisate.

Insbesondere betrifft die vorliegende Erfindung die Herstellung von Nitrilkautschuken durch radikalische Polymerisation, die in Lösung und in Gegenwart spezieller Regler-Verbindungen durchgeführt wird, mit nachfolgender Umsetzung mit Bismaleinimiden unter UV-Einstrahlung, sowie Nitrilkautschuke, die in der Polymer-Hauptkette oder an den Kettenenden auf die Regler-Verbindungen zurückgehende Strukturelemente aufweisen.

Unter Nitrilkautschuken, abgekürzt auch als "NBR" bezeichnet, werden Kautschuke verstanden, bei denen es sich um Co- oder Terpolymere aus mindestens einem α,β-ungesättigten Nitril, mindestens einem konjugierten Dien und gegebenenfalls einem oder mehreren weiteren copolymerisierbaren Monomeren handelt. Unter hydrierten Nitrilkautschuken ("HNBR") werden entsprechende Co- oder Terpolymere verstanden, bei denen die C=C-Doppelbindungen der einpolymerisierten Dien-Einheiten ganz oder teilweise hydriert sind.

Sowohl NBR als auch HNBR nehmen seit vielen Jahren einen festen Platz im Bereich der Spezialelastomere ein. Sie verfügen über ein ausgezeichnetes Eigenschaftsprofil in Form einer ausgezeichneten Ölbeständigkeit, einer guten Hitzebeständigkeit und einer hervorragenden Beständigkeit gegen Ozon und Chemikalien, wobei letztere im Fall des HNBR noch ausgeprägter als beim NBR ist. NBR und HNBR weisen ferner sehr gute mechanische sowie anwendungstechnische Eigenschaften auf. Aus diesem Grund finden sie breite Verwendung in den verschiedensten Anwendungsgebieten und werden beispielsweise eingesetzt zur Herstellung von Dichtungen, Schläuchen, Riemen und Dämpfungselementen im Automobilsektor, ferner für Statoren, Bohrlochdichtungen und Ventildichtungen im Bereich der Ölförderung sowie auch für zahlreiche Teile der Elektroindustrie, des Maschinen- und Schiffsbaus. Kommerziell erhältlich sind eine Vielzahl unterschiedlicher Typen, die sich je nach Anwendungsbereich durch unterschiedliche Monomere, Molekulargewichte, Polydispersitäten sowie mechanische und physikalische Eigenschaften auszeichnen. Neben den Standardtypen werden vor allem Spezialtypen, die sich durch Gehalte spezieller Termonomere oder besondere Funktionalisierungen auszeichnen, zunehmend nachgefragt.

Im praktischen Einsatz der (H)NBR Kautschuke kommt dabei auch der Vulkanisation der Kautschuke, d.h. insbesondere dem Vernetzersystem und den Vulkanisationsbedingungen, eine wachsende Bedeutung zu. So wurden neben den bereits seit vielen Jahrzehnten existierenden klassischen Kautschuk-Vernetzungssystemen auf Basis von Peroxiden bzw. Schwefel in den letzten Jahren diverse neue Konzepte zur alternativen Vernetzung entwickelt. Derartige Vernetzungskonzepte beziehen auch Polymere mit ein, die aufgrund funktioneller Gruppen nicht allen Vernetzungsformen und -agentien zugänglich sind und daher eine besondere Herausforderung darstellen.

Großtechnisch werden Nitrilkautschuke fast ausschließlich durch sogenannte Emulsionspolymerisation hergestellt. Zur Regelung des Molekulargewichts und damit auch der Viskosität des entstehenden Nitrilkautschuks werden dabei üblicherweise Dodecylmercaptane, insbesondere Tertiärdodecylmercaptane ("TDDM" oder auch "TDM" abgekürzt) eingesetzt. Nach der Polymerisation wird der erhaltene NBR-Latex in einem ersten Schritt koaguliert und daraus der NBR-Feststoff isoliert. Sofern eine weiterführende Hydrierung des Nitrilkautschuks zum HNBR gewünscht ist, erfolgt diese Hydrierung ebenfalls nach bekannten Methoden des Standes der Technik, beispielsweise unter Einsatz homogener oder auch heterogener Hydrierkatalysatoren. Die Katalysatoren basieren üblicherweise auf Rhodium, Ruthenium oder Titan. Es können aber auch Platin, Iridium, Palladium, Rhenium, Ruthenium, Osmium, Kobalt oder Kupfer entweder als Metall oder aber bevorzugt in Form von Metallverbindungen eingesetzt werden.

Es gab bereits verschiedenste Ansätze zur Optimierung der Herstellungsverfahren von NBR sowie HNBR. So wurde versucht, die Polymerisation zum Nitrilkautschuk in organischer Lösung durchzuführen. In der Zusammenfassung der Dissertation von C. Hollbeck, Universität-Gesamthochschule Essen, 1995, Seite II, wird für die Copolymerisation von Acrylnitril und 1,3-Butadien in organischer Lösung folgendes festgestellt (Zitat): "Mit einem Zahlenmittel des Polymerisationsgrades Pn von 1589 (Molekulargewicht (Mn)= ∼85.800 g/mol) und einem Umsatz von 40,5% konnten die gesteckten Ziele bei einer Reaktionstemperatur von 343 K in 40 Stunden realisiert werden. Eine Zeitverkürzung auf 18 Stunden ließ sich nur unter Reduzierung des geforderten Umsatzes erreichen. Wie Testversuche zeigten, ist unter den gegebenen Bedingungen auch bei einer Erhöhung der Temperatur auf 353 K eine Kombination von Pn ≥ 1400 und Umsatz größer als 40% nicht im Bereich des Möglichen". Eine Beschränkung des erreichbaren Umsatzes auf gut 40 % innerhalb einer Reaktionszeit von 40 Stunden machen das dort beschriebene Verfahren der organischen Lösungspolymerisation für die Praxis technisch und wirtschaftlich untauglich.

Die WO-A-2011/032832 beschreibt die Herstellung von Nitrilkautschuken durch radikalische Polymerisation in Gegenwart spezieller Regler-Verbindungen, die in der Polymer-Hauptkette oder an den betreffenden Enden als Strukturelemente erkennbar sind.

Ein entsprechendes Verfahren ist auch in Macromol. Rapid Commun., 2010, 31, 1616-21 beschrieben. Die lösungsbasierte RAFT-vermittelte Synthese von Acrylnitril-Butadien-Kautschuk (NBR) unter Verwendung von Trithiocarbonaten und Dithioestern führte zu Molekulargewichten von bis zu 60000 g/mol mit PDI-Werten von < 2,0 und einer Umsetzung von bis zu 50 % innerhalb von 9 Stunden Reaktionszeit.

Aus Polym. Chem., 2012, 3, 1048 ist ein Verfahren zur Herstellung eines über Bistriazolyl-Gruppen gekuppelten Nitrilkautschuks bekannt. Dabei wird ein Nitrilkautschuk, der kovalent gebundene Kohlenstoff-Kohlenstoff-Dreifachbindungen aufweist, mit einem Diazid so umgesetzt, dass sich Bistriazolyl-Gruppen bei der Kupplung ergeben. Zur Herstellung ist typischerweise ein metallhaltiger Katalysator, insbesondere ein kupferhaltiger Katalysator, notwendig.

Die beschriebenen Regler-Verbindungen sind aus der sogenannten RAFT-Technologie bekannt. Diese Technologie wird bereits zur Synthese verschiedener Polymere eingesetzt (WO-A-2001/60792, US 7,230,063 B1**,** WO-A-2007/003782, US-A-2008/0153982, WO-A-2005/061555).

Vor dem oben geschilderten Hintergrund bestand die Aufgabe der vorliegenden Erfindung somit einerseits darin, Nitrilkautschuke zur Verfügung zu stellen, die den Aufbau besonderer Polymerarchitekturen und Mikrostrukturen und damit die Einstellung besonderer Eigenschaftsprofile für die späteren Anwendungen ermöglichen und ferner eine einfache Vernetzung bzw. Kettenverlängerung zulassen. Zum anderen sollte gleichzeitig die Aufgabe gelöst werden, diese speziellen Nitrilkautschuke mit einer breiten Palette von Molekulargewichten und Polydispersitäten und über ein möglichst einfaches Herstellungsverfahren zugänglich zu machen. Weiterhin ist ein Verfahren zur Herstellung der Polymere aus wirtschaftlichen Gründen vorteilhaft, bei dem die Reaktionszeiten unter Beibehaltung der erzielten Molmassen minimiert werden können. Um die guten Alterungseigenschaften der Polymere zu erhalten, ist ein Verfahren von Interesse, bei dem keine Metalle als Katalysatoren verwendet werden, da diese sich, wie dem Fachmann bekannt ist, negativ in dieser Hinsicht auswirken können.

Es wurde gefunden, dass es möglich ist, Nitrilkautschuke durch radikalische Polymerisation in Lösung oder Emulsion unter Einsatz spezieller RAFT-Regler herzustellen, die über eine Tetrazolfunktionalisierung erlauben, die Kettenlängen durch polymeranaloge Kupplungsreaktionen zu erhöhen.

Die Aufgabe wird erfindungsgemäß gelöst durch ein Verfahren zur Herstellung eines über Bisdihydropyrazol-Gruppen gekuppelten Nitrilkautschuks durch Umsetzung eines Nitrilkautschuks, der auf konjugierten Dienen, α,β-ungesättigten Nitrilen und gegebenenfalls weiteren copolymerisierbaren Monomeren als Monomeren basiert, hydriert sein kann und kovalent gebundene Tetrazolgruppen aufweist, mit einer bifunktionalen En-Verbindung, in der die En-Gruppen mit den Tetrazolgruppen jeweils (über eine Nitrilimin-Zwischenstufe) zu Dihydropyrazol-Gruppen umsetzbar sind und zu diesen umgesetzt werden.

Der Begriff "über Bisdihydropyrazol-Gruppen gekuppelt" bedeutet eine Kupplung durch ein chemisches Strukturelement, das (mindestens) zwei Dihydropyrazol-Gruppen chemisch gebunden aufweist. Die Dihydropyrazol-Gruppen sind dabei in der Regel über einen organischen Rest kovalent miteinander verbunden.

Die Tetrazolgruppe liegt vorzugsweise als Rest der allgemeinen Formel (1) 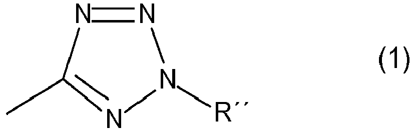 vor, worin R" einen, gegebenenfalls substituierten, Arylrest darstellt. Geeignete Arylreste sind beispielsweise Phenyl-, Naphthyl- oder Anthracenyl-Gruppen, die ohne Substituenten oder in substituierter Form vorliegen können. Mögliche Substituenten der Arylreste sind insbesondere C₁-C₁₂-Alkylreste, besonders bevorzugt C₁-C₆-Alkylgruppen, insbesondere C₁-C₃-Alkylgruppen. So kann beispielsweise ein Tolylrest vorliegen. Mögliche weitere Substituenten der Arylreste sind Amino-, Carboxy- oder Hydroxyfunktionen.

Dabei ist der zu kuppelnde Nitrilkautschuk vorzugsweise erhältlich durch radikalische Polymerisation mindestens eines konjugierten Diens, mindestens eines α,β-ungesättigten Nitrils und gegebenenfalls eines oder mehrerer weiterer copolymerisierbarer Monomere, in Gegenwart mindestens eines organischen Lösungsmittels und mindestens eines Reglers, wobei als Regler mindestens eine Verbindung der allgemeinen Formel (I) 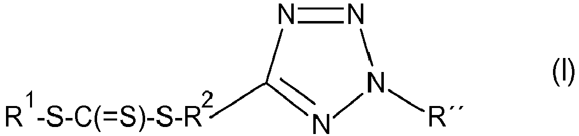 mit der Bedeutung
- R¹: Kohlenwasserstoffrest, der substituiert sein kann und nach homolytischer Spaltung der R¹-S-Bindung ein primäres Radikal ausbildet,
- R²: Kohlenwasserstoffrest, der substituiert sein kann, eine oder mehrere Carboxylgruppen enthalten kann und nach homolytischer Spaltung der S-R²-Bindung ein sekundäres, tertiäres oder aromatisch stabilisiertes Radikal ausbildet,
- R": wie vorstehend angegeben,
eingesetzt wird.

Vorzugsweise ist dabei in der Verbindung der allgemeinen Formel (I) R¹ein C₈-C₁₅-Alkylrest und R² ein Rest -CHR³-C(=O)OR⁴- mit R³ C₁-C₃-Alkyl und R⁴ C₁-C₆-Alkylen, das durch Carboxyl-und/oder Arylgruppen unterbrochen sein kann, und R" Phenyl, Tolyl, Naphthyl oder Anthracenyl ist.

R⁴ kann ein C₁-C₆-Allcylen-Rest sein, der linear oder verzweigt sein kann und durch Carboxyl-und/oder Arylgruppen unterbrochen sein kann. Die Carboxyl- und/oder Arylgruppen können dabei auch endständig vorliegen, so dass der Rest R⁴ über eine Carboxyl- und/oder Arylgruppe mit der Tetrazolgruppe verbunden ist.

Vorzugsweise ist der Rest R⁴ über eine derartige Arylgruppe mit der Tetrazolgruppe verbunden. Besonders bevorzugt ist diese Arylgruppe über eine Carboxylgruppe mit dem Rest des Restes R⁴ verbunden. Bevorzugt handelt es sich beim Rest R⁴ um einen Rest C₁-C₆-Alkylen-O-C(=O)-Arylen-. Dabei ist Arylen vorzugsweise 1,4-Phenylen. Besonders bevorzugt für R⁴ ist dabei C₁₋₆-Alkylen, ganz besonders bevorzugt 1,3-Propylen.

Vorzugsweise sind in der bifunktionalen En-Verbindung zwei En-Gruppen der allgemeinen Formel

R'-X-CH=CH-X-R' (II)

vorliegend, in der X jeweils unabhängig einen Rest C=O, CHOH, CHI, CHBr, CHCl, CHNO₂, CHNH₂, CHCOOH, CHC₆H₅, CHCN bedeutet und R' jeweils unabhängig einen C₁-C₁₂-Kohlenwasserstoffrest bedeutet, der ein oder mehrere Heteroatome enthalten kann, wobei die beiden Reste R' an einer En-Gruppe der allgemeinen Formel (II) zu einem Ring verbunden sein können und die beiden En-Gruppen der allgemeinen Formel (II) über jeweils mindestens einen der Reste R' kovalent miteinander verbunden sind.

Vorzugsweise weist die bifunktionale En-Verbindung zwei Maleinimid-Gruppen auf, deren Stickstoffatome über einen C₁-C₁₂-Alkylenrest, der substituiert und von Heteroatomen und/oder Arylgruppen unterbrochen sein kann, miteinander verbunden sind.

Bevorzugt sind die endständigen Stickstoffatome über einen linearen C₄-C₈-Alkylenrest miteinander verbunden.

Alternativ zur vorstehenden Herstellung ist der zu kuppelnde Nitrilkautschuk gemäß einer Ausführungsform der Erfindung erhältlich durch
a) radikalische Polymerisation mindestens eines konjugierten Diens, mindestens eines α,β-ungesättigten Nitrils und gegebenenfalls eines oder mehrerer weiterer copolymerisierbarer Monomere, vorzugsweise in Gegenwart mindestens eines organischen Lösungsmittels, und in Gegenwart mindestens eines Reglers und
b) optional anschließend eine Hydrierung,
   wobei als Regler in Schritt a) mindestens eine Verbindung der allgemeinen Strukturformel (VI) eingesetzt wird, 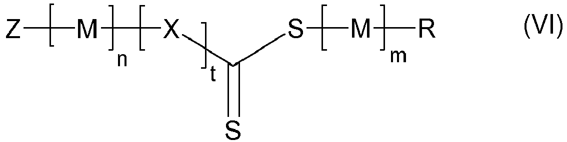
worin
- Z: für H, einen linearen oder verzweigten, gesättigten, ein- oder mehrfach ungesättigten Alkylrest, einen gesättigten, ein- oder mehrfach ungesättigten Carbo- oder Heterocyclylrest, Aryl, Heteroaryl, Arylalkyl, Heteroarylalkyl, Alkoxy, Aryloxy, Heteroaryloxy, Amino, Amido, Hydroxyimino, Carbamoyl, Alkoxycarbonyl, F, Cl, Br, I, Hydroxy, Phosphonato, Phosphinato, Alkylthio, Arylthio, Sulfanyl, Thiocarboxy, Sulfinyl, Sulfono, Sulfino, Sulfeno, Sulfonsäuren, Sulfamoyl, Silyl, Silyloxy, Nitril, Carbonyl, Carboxy, Oxycarbonyl, Oxysulfonyl, Oxo, Thioxo, Borate, Selenate, Epoxy, Cyanate, Thiocyanate, Isocyanate, Thioisocyanate und Isocyanide,
- R: **(a)** für den Fall, dass m ≠ 0 ist, die gleichen Bedeutungen besitzt wie der Rest Z und **(b)** für den Fall, dass m = 0 ist, für H, einen linearen oder verzweigten, gesättigten, ein-oder mehrfach ungesättigten Alkylrest, einen gesättigten, ein- oder mehrfach ungesättigten Carbo- oder Heterocyclylrest, Aryl, Heteroaryl, Arylalkyl, Heteroarylalkyl, Alkoxy, Aryloxy, Heteroaryloxy, Amino, Amido, Carbamoyl, Alkoxy, Aryloxy, Alkylthio, Arylthio, Sulfanyl, Thiocarboxy, Sulfinyl, Sulfono, Sulfino, Sulfeno, Sulfonsäuren, Sulfamoyl, Carbonyl, Carboxy, Oxycarbonyl, Oxysulfonyl, Oxo, Thioxo, Epoxy, Cyanate, Thiocyanate, Isocyanate, Thioisocyanate oder Isocyanide steht,
- M: für Wiederholungseinheiten eines oder mehrerer, ein- oder mehrfach ungesättigter Monomeren steht, umfassend konjugierte oder nicht-konjugierte Diene, Alkine und Vinylverbindungen, oder für ein Strukturelement, welches sich ableitet Polymeren umfassend Polyether, insbesondere Polyalkylenglykolether und Polyalkylenoxide, Polysiloxane, Polyole, Polycarbonate, Polyurethane, Polyisocyanate, Polysaccharide, Polyester und Polyamide,
- n und m: gleich oder verschieden sind und jeweils im Bereich von 0 bis 10.000 liegen,
- t: 0 oder 1 ist, sofern n= 0, und gleich 1 ist, sofern n ≠ 0, und
- X: für C(Z₂), N(Z), P(Z), P(=O)(Z), O, S, S(=O) oder S(=O)₂ steht, wobei Z in diesen Resten die gleichen Bedeutungen besitzt, wie zuvor für die Formel (VI) ausgeführt,

und nachfolgende Umsetzung des so erhaltenen Nitrilkautschuks mit einer Verbindung, die die kovalente Anbindung einer Tetrazolgruppe, vorzugsweise eines Restes der allgemeinen Formel (1), in dem R" eine beliebige Arylgruppe darstellt, erlaubt und zu dieser Anbindung führt.

**Die Erfindung betrifft** ferner einen über Dihydropyrazol-Gruppen gekuppelten Nitrilkautschuk, der vorzugsweise nach dem vorstehenden Verfahren erhältlich ist.

**Die Erfindung betrifft** zudem die Verwendung des über Dihydropyrazol-Gruppen gekuppelten Nitrilkautschuks zur Herstellung von Formkörpern, Überzügen oder Vulkanisaten.

**Die Erfindung betrifft** zudem vulkanisierbare Mischungen, enthaltend den vorstehend beschriebenen Nitrilkautschuk, mindestens einen Vernetzer, optional mindestens einen Füllstoff und optional einen oder mehrere weitere Kautschuk-Additive.

**Die Erfindung betrifft** zudem ein Verfahren zur Herstellung von Vulkanisaten, bei denen die beschriebene vulkanisierbare Mischung einer Vernetzung unterzogen wird, bevorzugt durch Zusatz mindestens eines Vernetzers oder durch photochemische Aktivierung.

**Die Erfindung betrifft** ferner Vulkanisate, bevorzugt Formteile, die nach dem vorstehenden Verfahren erhältlich sind.

**Die Erfindung betrifft** ferner einen Regler der allgemeinen Formel (I), wie er vorstehend definiert ist.

**Die Erfindung betrifft** ferner einen Nitrilkautschuk, der Tetrazol-Gruppen aufweist und zur Kupplung mit En-Gruppen unter UV-Einstrahlung geeignet ist, wie er vorstehend definiert ist.

Mit dem erfindungsgemäßen Verfahren gelingt es, in Zeiträumen, die denen der herkömmlichen Emulsionspolymerisation zur NBR-Herstellung vergleichbar sind, zu Umsätzen zu gelangen, die das Verfahren für eine großtechnische Umsetzung geeignet machen. So lässt sich bei einer Polymerisationszeit von kleiner 10 Stunden bereits ein Umsatz von 50% erzielen bei gleichzeitiger Erzeugung technisch akzeptabler Molekulargewichte (Mn > 50.000 g/mol) und mit - im Vergleich zu herkömmlichem Emulsions-NBR - bis heute unerreicht niedrigen Polydispersitäten von deutlich kleiner 2.0.

Es wurde erfindungsgemäß gefunden, dass Nitrilkautschuke, die Tetrazolfunktionen aufweisen, mit organischen Verbindungen, die mindestens zwei En-gruppen aufweisen, wie Bismaleinimiden, unter Ausbildung von Dihydropyrazol-Gruppen gekuppelt werden können.

Bevorzugt werden dabei Nitrilkautschuke durch radikalische Polymerisation der Ausgangsmonomere in Gegenwart eines Reglers erhalten, der eine Tetrazolgruppe enthält. Besonders bevorzugt werden dazu Tetrazol-funktionalisierte Trithiocarbonate eingesetzt.

Durch den Einsatz dieser Tetrazol-funktionalisierten Trithiocarbonate als RAFT-Regler war es möglich, α-funktionalisierte NBR-Bausteine zu erhalten. Bevorzugt erfolgte die Herstellung in Gegenwart von Azo-Initiatoren in organischen Lösungsmitteln wie Chlorbenzol oder Aceton. Diese α-funktionalisierten NBR-Bausteine konnten dann in der bevorzugt UV-induzierten Tetrazol-En-Kupplungsreaktion mit Verknüpfungsreagenzien wie 1,6-Bis(maleimido)hexan eingesetzt werden. Die Polymer-Polymer-Kupplung führte zu linearen Polymeren mit Molekulargewichten im Bereich von insbesondere 8900 g/mol bis 94000 g/mol und Polydispersitäten im Bereich von insbesondere 1,3 bis 1,7.

Durch die UV-induzierte Tetrazol-En-Reaktion ist es möglich, die Kettenlänge der NBR-Kautschuke deutlich zu vergrößern und zu hohen Molekulargewichten zu gelangen.

Im Sinne dieser Anmeldung ist der Begriff "Nitrilkautschuk(e)" breit zu interpretieren und umfasst sowohl die Nitrilkautschuke als auch hydrierte Nitrilkautschuke. Sofern es sich um hydrierte Nitrilkautschuke handelt, bedeutet die oben genannte Formulierung "Nitrilkautschuke enthaltend Wiederholungseinheiten abgeleitet von" somit, dass es sich bei den Wiederholungseinheiten, die auf das konjugierte Dien zurückgehen, um solche handelt, in denen die nach der Polymerisation im Polymer zunächst vorhandenen C=C Doppelbindungen ganz oder teilweise hydriert sind.

Soweit in dieser Anmeldung der Begriff "substituiert" verwendet wird, so bedeutet dies, dass ein Wasserstoff-Atom an einem angegebenen Rest oder Atom durch eine der angegebenen Gruppen ersetzt ist, mit der Maßgabe, dass die Wertigkeit des angegebenen Atoms nicht überschritten wird und immer nur unter der Bedingung, dass diese Substitution zu einer chemisch stabilen Verbindung führt.

In der difunktionalen En-Verbindung sind (in den En-Gruppen der allgemeinen Formel (II)) mögliche Substituenten derart, dass sie die Umsetzung der difunktionalen En-Verbindung mit dem Tetrazolrest in der Kupplungsreaktion nicht behindern. Beispiele möglicher Substituenten sind vorstehend angegeben.

Die En-Verbindung kann auch durch weitere En-Gruppen substituiert sein. Hierdurch werden polyenfunktionale Verbindungen erhalten, die mehr als zwei En-Funktionalitäten tragen. Es handelt sich damit um organische Verbindungen, die mindestens zwei En-Funktionalitäten tragen.

Auch in den bevorzugt als Regler eingesetzten Verbindungen der allgemeinen Formel (I) können die Reste R¹und R² sowie R" substituiert sein. Auch diese Substituenten sind so gewählt, dass sie die Tetrazol-En-Reaktion und die chemische Stabilität der Verbindung der allgemeinen Formel (I) nicht einschränken. Bevorzugt sind Substituenten der Reste R¹und R² sowie R" C₁-C₆-Alkyl-Reste.

Bevorzugt liegen in der difunktionalen En-Verbindung elektronenziehende Substituenten vor, vorzugsweise ausgewählt aus OH, =O, -I, -Br, -Cl, -NO₂, -NH₂, -COOH, -C₆H₅, -CN.

Der NBR, der eine kovalent gebundene Tetrazolfunktion aufweist, enthält eine Tetrazolfunktion, die zur Umsetzung in der beschriebenen Kupplungsreaktion geeignet ist. Ihre sterische Anordnung und mögliche Anbindungsgruppen sind so gewählt, dass die UV-aktivierte Kupplungsreaktion unterstützt wird.

Besonders bevorzugt ist die Tetrazol-Gruppe endständig am Kettenende oder in einer Seitenkette des NBR kovalent angebunden und weist die Struktur der allgemeinen Formel (1) auf, wobei R" Benzyl- oder ein beliebiger Aromat ist. Bevorzugt ist R" Phenyl oder Benzyl.

In der Umsetzung zwischen En-Funktionalitäten der mindestens difunktionalen En-Verbindung und Tetrazol-Funktionalitäten wird vorzugsweise ein solches Mengenverhältnis eingestellt, das einem Mol-Verhältnis zwischen En-Funktionalitäten und Tetrazol-Funktionalitäten von 1:1 möglichst nahe kommt. Vorzugsweise beträgt das Mol-Verhältnis 1:0,5 bis 0,5:1, besonders bevorzugt 1:0,8 bis 0,8:1, insbesondere 0,94 : 1 bis 1 : 0,94, speziell 0,99 : 1 bis 1 : 0,99.

Je genauer die Mengen an Tetrazol-Gruppen und En-Funktionalitäten aufeinander abgestimmt sind, umso höhere Molekulargewichte sind zugänglich. Bei einer stärkeren Abweichung von dem äquimolaren Verhältnis kommt es zu einer breiteren Molekulargewichtsverteilung.

Die Umsetzung wird vorzugsweise in einem organischen Lösungsmittel durchgeführt, besonders bevorzugt in einem organischen polaren Lösungsmittel, das NBR löst. Besonders bevorzugte Lösungsmittel sind DMF, Pyridin, Methylenchlorid, Acetonitril oder Aceton, sowie die weiteren nachstehend bei der NBR-Herstellung genannten Lösungsmittel.

Sofern nach der Bildung des Dihydropyrazols eine Hydrierung des NBR durchgeführt wird, wird bevorzugt das identische Lösungsmittel für die Kupplung wie für die Hydrierung verwendet.

Zur Kupplung liegt der NBR vorzugsweise in einer Konzentration von 0.01 bis 100 g/l, besonders bevorzugt 0.1 bis 60 g/l, insbesondere 0.6 bis 20 g/l vor.

Die Kupplung kann allgemein in breiten Konzentrationsbereichen und bei beliebiger geeigneter Temperatur erfolgen. Vorzugsweise wird die Kupplung bei einer Temperatur im Bereich von 0 bis 200 °C, besonders bevorzugt 20 bis 140 °C, insbesondere etwa Raumtemperatur (22-28°C) durchgeführt. Dabei kann die Kupplung in der Regel an Luft erfolgen.

Die Kupplung erfolgt bevorzugt unter Einstrahlung elektromagnetischer Wellen. Diese ermöglichen die Abwesenheit eines metallischen Katalysators. Aufgrund der Anwendungseigenschaften ist es vorteilhaft, die Kupplung metallfrei durchzuführen, da Metallgehalte die Anwendungseigenschaften des erhaltenen NBR verschlechtern können.

Die Lösung des tetrazolfunktionalen NBRs und des En-funktionalen Kupplungsreagenzes wird einer elektromagnetischen Strahlung ausgesetzt. Bevorzugt hat diese Strahlung eine Wellenlänge im Bereich von 10 bis 800 nm, besonders bevorzugt 100 bis 400 nm. Ganz besonders bevorzugt liegt diese im Bereich von 200 bis 380 nm.

Das erfindungsgemäße Verfahren erlaubt es, in kurzen Reaktionszeiten hohe Molekulargewichte des NBR zu erreichen, gekoppelt mit einem engen PDI. Bevorzugt weisen die über Dihydropyrazol-Gruppen gekuppelten Nitrilkautschuke ein Molekulargewicht (M_{w}) im Bereich von 2.000 bis 500.000 g/mol, besonders bevorzugt 5.000 bis 400.000 g/mol auf. Der Polydispersitätsindex (PDI) beträgt dabei vorzugsweise weniger als 2,0, besonders bevorzugt 1,7 oder weniger, insbesondere 1,3 bis 1,7.

Der zur Kupplung eingesetzte Nitrilkautschuk, der kovalent gebundene Tetrazol-Funktionalitäten aufweist, kann nach unterschiedlichen Verfahren erhalten werden. Beispielsweise kann zunächst der nicht funktionalisierte Nitrilkautschuk hergestellt werden unter Verwendung eines bekannten RAFT-Reglers, und die Anbindung des Restes der allgemeinen Formel (1) erfolgt nachfolgend durch Umsetzung mit einer geeigneten Verbindung, die diese Gruppierung aufweist und eine Anbindung an den Nitrilkautschuk erlaubt. Diese Form der Umsetzung kann nach dem Fachmann geläufigen Bedingungen durchgeführt werden. Alternativ kann die Tetrazol-Funktionalität durch dem Fachmann geläufige Methoden in den RAFT-Regler eingeführt werden, der dann zur Einstellung des gewünschten Molekulargewichtes während der Polymerisation eingesetzt und so in die Polymerkette eingebaut wird.

Bei der erstgenannten Methode erfolgt zunächst die Herstellung von Nitrilkautschuken, indem man
a) eine radikalische Polymerisation mindestens eines konjugierten Diens, mindestens eines α,β-ungesättigten Nitrils und gegebenenfalls eines oder mehrerer weiterer copolymerisierbarer Monomere in Gegenwart mindestens eines organischen Lösungsmittels und mindestens eines Reglers durchführt und
b) optional anschließend eine Hydrierung,
   wobei als Regler mindestens eine Verbindung der allgemeinen Strukturformel (VI) eingesetzt wird, 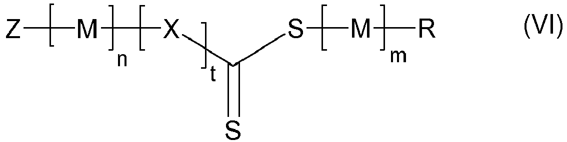
worin
- **Z**: für H, einen linearen oder verzweigten, gesättigten, ein- oder mehrfach ungesättigten Alkylrest, einen gesättigten, ein- oder mehrfach ungesättigten Carbo- oder Heterocyclylrest, Aryl, Heteroaryl, Arylalkyl, Heteroarylalkyl, Alkoxy, Aryloxy, Heteroaryloxy, Amino, Amido, Hydroxyimino, Carbamoyl, Alkoxycarbonyl, F, Cl, Br, I, Hydroxy, Phosphonato, Phosphinato, Alkylthio, Arylthio, Sulfanyl, Thiocarboxy, Sulfinyl, Sulfono, Sulfino, Sulfeno, Sulfonsäuren, Sulfamoyl, Silyl, Silyloxy, Nitril, Carbonyl, Carboxy, Oxycarbonyl, Oxysulfonyl, Oxo, Thioxo, Borate, Selenate, Epoxy, Cyanate, Thiocyanate, Isocyanate, Thioisocyanate und Isocyanide,
- R: (a) für den Fall, dass m ≠ 0 ist, die gleichen Bedeutungen besitzen kann wie der Rest Z und (b) für den Fall, dass m = 0 ist, für H, einen linearen oder verzweigten, gesättigten, ein- oder mehrfach ungesättigten Alkylrest, einen gesättigten, ein- oder mehrfach ungesättigten Carbo-oder Heterocyclylrest, Aryl, Heteroaryl, Arylalkyl, Heteroarylalkyl, Alkoxy, Aryloxy, Heteroaryloxy, Amino, Amido, Carbamoyl, Alkoxy, Aryloxy, Alkylthio, Arylthio, Sulfanyl, Thiocarboxy, Sulfinyl, Sulfono, Sulfino, Sulfeno, Sulfonsäuren, Sulfamoyl, Carbonyl, Carboxy, Oxycarbonyl, Oxysulfonyl, Oxo, Thioxo, Epoxy, Cyanate, Thiocyanate, Isocyanate, Thioisocyanate oder Isocyanide steht,
- M: für Wiederholungseinheiten eines oder mehrerer, ein- oder mehrfach ungesättigter Monomeren steht, umfassend konjugierte oder nicht-konjugierte Diene, Alkine und Vinylverbindungen, oder für ein Strukturelement, welches sich ableitet Polymeren umfassend Polyether, insbesondere Polyalkylenglykolether und Polyalkylenoxide, Polysiloxane, Polyole, Polycarbonate, Polyurethane, Polyisocyanate, Polysaccharide, Polyester und Polyamide,
- n und m: gleich oder verschieden sind und jeweils im Bereich von 0 bis 10.000 liegen,
- t: 0 oder 1 ist, sofern n= 0, und gleich 1 ist, sofern n ≠ 0, und
- X: für C(Z₂), N(Z), P(Z), P(=O)(Z), O, S, S(=O) oder S(=O)₂ steht, wobei Z in diesen Resten die gleiche Bedeutungen besitzen kann, wie zuvor für die Formel (VI) ausgeführt.

Die erfindungsgemäßen, optional hydrierten Nitrilkautschuke zeichnen sich durch die Anwesenheit von ein oder mehreren Strukturelementen der allgemeinen Formeln (I) oder (VI) entweder in der Polymer-Hauptkette oder als Endgruppen aus.

Die in den Resten Z und R der allgemeinen Formel (VI) genannten Bedeutungen können jeweils ein-oder mehrfach substituiert sein. Bevorzugt weisen die folgenden Reste eine ein- oder mehrfache Substitution auf: Alkyl, Carbocyclyl, Heterocyclyl, Aryl, Heteroaryl, Arylalkyl, Heteroarylalkyl, Alkoxy, Aryloxy, Alkylthio, Arylthio, Amino, Amido, Carbamoyl, Phosphonato, Phosphinato, Sulfanyl, Thiocarboxy, Sulfinyl, Sulfono, Sulfino, Sulfeno, Sulfamoyl, Silyl, Silyloxy, Carbonyl, Carboxy, Oxycarbonyl, Oxysulfonyl, Oxo, Thioxo, Borate, Selenate und Epoxy.

Als Substituenten kommen wiederum - soweit sich chemisch stabile Verbindungen ergeben - alle Bedeutungen in Frage, die Z annehmen kann. Besonders geeignet als Substituenten sind Halogen, bevorzugt Fluor, Chlor, Brom oder Iod, Nitril (CN) und Carboxy.

Die für Z und R in der allgemeinen Formel (VI) genannten Bedeutungen schließen explizit auch Salze der genannten Reste ein, soweit diese chemisch möglich und stabil sind. Hierbei kann es sich beispielsweise um Ammonium-Salze, Alkali-Salze, Erdalkali-Salze, Aluminium-Salze oder um protonierte Formen der Regler der allgemeinen Formel (VI) handeln.

Die für Z und R in der allgemeinen Formel (VI) genannten Bedeutungen schließen auch organometallische Reste ein, beispielsweise solche, die dem Regler eine Grignard Funktion verleihen. Z und R können ferner ein Carbanion darstellen bzw. aufweisen mit Lithium, Zink, Zinn, Aluminium, Blei und Bor als Gegen-Ion.

Es ist ferner möglich, dass der Regler über einen Linker an eine Festphase oder Trägersubstanz angekoppelt ist. Bei dem Linker kann es sich um dem Fachmann bekannten Wang-, Sasrin-, Rink-Säure-, 2-Chlortrityl-, Mannich-, Safety-Catch-, Traceless- oder photolabile Linker handeln. Als Festphasen oder Trägersubstanzen kommen beispielsweise Silica, Ionenaustauscher-harze, Tone (Clay), Montmorillonite, vernetztes Polystyrol, Polyethylenglykol gepfropft auf Polystyrol, Polyacrylamide ("Pepsyn"), Polyethylenglykol-Acrylamid-Copolymere (PEGA), Cellulose, Baumwolle und gekörntes poröses Glas (CPG, controlled pore glass) in Frage.

Es ist ferner möglich, dass die Regler der allgemeinen Formel (VI) als Liganden für metallorganische Komplexverbindungen fungieren, z.B. für solche auf Basis der Zentralmetalle Rhodium, Ruthenium, Titan, Platin, Iridium, Palladium, Rhenium, Ruthenium, Osmium, Kobalt, Eisen oder Kupfer.

Die in der oben genannten allgemeinen Formel (VI) für den Rest "M" aufgeführten Bedeutungen können ein- oder mehrfach substituiert sein. Somit kann es sich bei M um Wiederholungseinheiten eines oder mehrerer, einfach oder mehrfach ungesättigter Monomere handeln, bevorzugt um optional ein- oder mehrfach substituierte konjugierte oder nicht-konjugierte Diene, optional ein- oder mehrfach substituierte Alkine oder optional ein- oder mehrfach substituierte Vinylverbindungen, beispielsweise fluorierte ein- oder mehrfach ungesättigte Vinylverbindungen, oder aber um ein divalentes Strukturelement, welches sich ableitet von substituierten oder unsubstituierten Polymeren umfassend Polyether, insbesondere Polyalkylenglykolether und Polyalkylenoxide, Polysiloxane, Polyole, Polycarbonate, Polyurethane, Polyisocyanate, Polysaccharide, Polyester und Polyamide. Hinter diesen Resten "M" kann sich somit ein monomerer oder polymerer Rest verbergen.

Bevorzugt wird ein Regler der allgemeinen Formel (VI) eingesetzt, bei dem
- Z und R: die zuvor für die allgemeine Formel (VI) genannten Bedeutungen besitzen und
- n, m und t: alle gleich Null sind.

Dieser bevorzugte Regler besitzt somit die allgemeine Struktur (VIa): 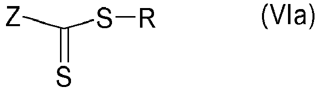 bei dem die Reste Z und R alle zuvor für die allgemeine Formel (VI) genannten Bedeutungen aufweisen können.

### Trithiocarbonate:

Als weiterer bevorzugter Regler kann ein Regler der allgemeinen Formel (VIb) eingesetzt werden, 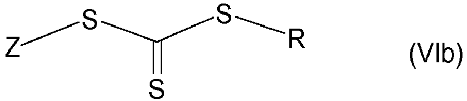 bei dem
- Z: die zuvor für die allgemeine Formel (VI) genannten Bedeutungen besitzt,
- R: die zuvor für die allgemeine Formel (VI) für die Variante b) mit m=0 genannten Bedeutungen besitzt, allerdings mit der Einschränkung, dass R nach homolytischer Spaltung der S-R Bindung entweder ein sekundäres, tertiäres oder aromatisch stabilisiertes Radikal ausbildet.

Dieser besonders bevorzugte Regler der allgemeinen Formel (VIb) ergibt sich aus dem Regler der allgemeinen Formel (VI), indem
n und m jeweils = 0 sind,
- t: gleich 1 ist,
- X: für Schwefel steht,
- Z: die zuvor für die allgemeine Formel (VI) genannten Bedeutungen besitzt und
- R: die zuvor für die allgemeine Formel (VI) für die Variante b) mit m=0 genannten Bedeutungen besitzt, allerdings mit der Einschränkung, dass R nach homolytischer Spaltung der S-R Bindung entweder ein sekundäres, tertiäres oder aromatisch stabilisiertes Radikal ausbildet.

Bei diesen besonders bevorzugten Reglern der allgemeinen Formel (VIb) handelt es sich somit in Abhängigkeit davon, ob Z und R im Rahmen der vorgegebenen Bedeutungen identisch sind oder nicht, um symmetrische oder asymmetrische Trithiocarbonate.

Besonders bevorzugt wird ein Regler der allgemeinen Formel (VIb) eingesetzt, bei dem
Z die zuvor für die allgemeine Formel (VI) genannten Bedeutungen besitzt und
R mit der Maßgabe, dass R nach homolytischer Spaltung der S-R Bindung entweder ein sekundäres, tertiäres oder aromatisch stabilisiertes Radikal ausbildet,
- für einen linearen oder verzweigten, gesättigten oder ein- oder mehrfach ungesättigten, optional ein- oder mehrfach substituierten Alkylrest steht, bevorzugt für einen entsprechenden C₃-C₂₀-Alkylrest, insbesondere für sec.-Butyl, tert.-Butyl, iso-Propyl, 1-Buten-3-yl, 2-Chloro-1-buten-2-yl, Propionsaeure-2-yl, Propionitril-2-yl, 2-Methylpropannitril-2-yl, 2-Methylpropionsaeure-2-yl oder 1H,1H,2-Keto-3-oxo-4H,4H,5H,5H-perfluorundecanyl, oder
- für einen gesättigten oder ein- oder mehrfach ungesättigten, optional ein- oder mehrfach substituierten Carbo- oder Heterocyclylrest steht, inbesondere für Cyclohexyl, Cumyl oder Cyclohexan-1-nitril-1-yl,
- für einen (Hetero)Arylrest steht, ganz besonders bevorzugt für einen C₆-C₂₄-(Hetero)arylrest, inbesondere für Phenyl, Pyridinyl oder Anthracenyl,
- für einen (Hetero)Aralkylrest steht, ganz besonders bevorzugt für Benzyl, Phenylethyl oder 1-Methyl-1-phenyleth-2-yl, oder
- für Thiocarboxy, Carbonyl, Carboxy, Oxo, Thioxo, Epoxy, sowie Salze der zuvor genannten Verbindungen steht.

Insbesondere bevorzugt wird ferner ein Regler der allgemeinen Formel (VIb) eingesetzt, bei dem
- Z: die zuvor für die allgemeine Formel (VI) genannten Bedeutungen besitzt, allerdings ebenfalls mit der zusätzlichen Einschränkung auf solche Bedeutungen, dass Z nach homolytischer Spaltung der Z-S Bindung entweder ein sekundäres, tertiäres oder aromatisch stabilisiertes Radikal ausbildet.

Hierbei handelt es sich dann um einen Trithiocarbonat-Regler, bei dem beide Reste R und Z polymerisationsinitüerend wirken.

Ganz besonders bevorzugt wird ferner ein Regler der allgemeinen Formel (VIb) eingesetzt, bei dem
R und Z gleich oder verschieden sind und mit der Maßgabe, dass R und Z nach homolytischer Spaltung der R-S bzw. Z-S Bindung jeweils ein sekundäres, tertiäres oder aromatisch stabilisiertes Radikal ausbilden,
- für einen linearen oder verzweigten, gesättigten oder ein- oder mehrfach ungesättigten, optional ein- oder mehrfach substituierten Alkylrest steht, bevorzugt für einen entsprechenden C₃-C₂₀-Alkylrest, insbesondere für sec.-Butyl, tert.-Butyl, iso-Propyl, 1-Buten-3-yl, 2-Chloro-1-buten-2-yl, Propionsaeure-2-yl, Propionitril-2-yl, 2-Methylpropannitril-2-yl, 2-Methylpropionsaeure-2-yl oder 1H,1H,2-Keto-3-oxo-4H,4H,5H,5H-perfluorundecanyl, oder
- für einen gesättigten oder ein- oder mehrfach ungesättigten, optional ein- oder mehrfach substituierten Carbo- oder Heterocyclylrest steht, inbesondere für Cyclohexyl, Cumyl oder Cyclohexan-1-nitril-1-yl,
- für einen (Hetero)Arylrest steht, ganz besonders bevorzugt für einen C₆-C₂₄-(Hetero)arylrest, inbesondere für Phenyl, Pyridinyl oder Anthracenyl,
- für einen (Hetero)Aralkylrest steht, ganz besonders bevorzugt für Benzyl, Phenylethyl oder 1-Methyl-1-phenyleth-2-yl, oder
- für Thiocarboxy, Carbonyl, Carboxy, Oxo, Thioxo, Epoxy, sowie Salze der zuvor genannten Verbindungen stehen.

Bezüglich der für die allgemeine Formel (VIb) und nachfolgend für die allgemeinen Formeln (VIc), (VId) und (VIe) verwendeten Formulierungen, "dass R nach homolytischer Spaltung der R-S-Bindung ein sekundäres oder tertiäres Radikal bildet", gelten die nachfolgenden Definitionen. Diese gelten ebenfalls in analoger Form für die entsprechenden Formulierung "dass Z nach homolytischer Spaltung der Z-S-Bindung ein sekundäres oder tertiäres Radikal bildet", soweit diese im Rahmen der Anmeldung im Zusammenhang mit Z verwendet wird.

Das Atom im Rest R, welches die Bindung an S in der allgemeinen Formel (VIb) bewirkt (bzw. den noch folgenden allgemeinen Formeln (VIc), (VId) und (VIe)), führt bei homolytischer Spaltung der R-S Bindung dann zu einem als "tertiär" zu bezeichnenden Radikal, wenn an dieses Atom (die Bindung zum Schwefel ausgenommen) mindestens
(i) über Einfachbindungen drei Substituenten oder
(ii) über eine Einfachbindung ein Substituent und über eine Doppelbindung ein weiterer Substituent gebunden sind oder
(iii) über eine Dreifachbindung ein Substituent gebunden ist,
   wobei alle vorgenannten Substituenten ungleich Wasserstoff sein müssen.

Das Atom im Rest R, welches die Bindung an S in den allgemeinen Formeln (VIb), (VIc), (VId) und (VIe) bewirkt, führt bei homolytischer Spaltung der R-S Bindung dann zu einem als "sekundär" zu bezeichnenden Radikal, wenn an dieses Atom
(i) über Einfachbindungen zwei Substituenten gebunden sind oder
(ii) über eine Doppelbindung ein Substituent gebunden ist,
wobei alle vorgenannten Substituenten ungleich Wasserstoff sein müssen und alle weiteren möglichen Substituenten H sind.

Beispiele für Reste R bzw. Z, die bei homolytischer Spaltung der R-S (bzw. Z-S) Bindung zu einem als "tertiär" zu bezeichnenden Radikal führen, sind z.B. tert. Butyl, Cyclohexan-1-nitril-1-yl und 2-Methylpropannitril-2-yl.

Beispiele für Reste R bzw. Z, die bei homolytischer Spaltung der R-S (bzw. Z-S) Bindung zu einem als "sekundär" zu bezeichnenden Radikal führen, sind z.B. sek-Butyl, iso-Propyl und Cycloalkyl, bevorzugt Cyclohexyl.

Bezüglich der nachfolgend für die Formel (VId) verwendeten Maßgabe, "dass Z nach homolytischer Spaltung der Z-S-Bindung ein primäres Radikal bildet", gilt folgende Definition: Das Atom im Rest Z, welches die Bindung an S in der allgemeinen Formel (VId) bewirkt, führt bei homolytischer Spaltung der Z-S Bindung dann zu einem als "primär" zu bezeichnenden Radikal, wenn an dieses Atom über eine Einfachbindung kein oder maximal ein Substituent gebunden ist, der nicht Wasserstoff ist. Für Z = H gilt die og. Maßgabe per Definition als erfüllt.

Beispiele für Reste Z, die bei homolytischer Spaltung der Z-S Bindung zu einem als "primär" zu bezeichnenden Radikal führen, sind somit z.B. H, lineare C₁-C₂₀ Alkylreste, OH, SH, SR und C₂-C₂₀ Alkylreste mit Verzweigungen jenseits des C-Atoms, das die Bindung an S bewirkt.

### Dithioester:

Als weiterer bevorzugter Regler kann ein Regler der allgemeinen Formel (VIc) eingesetzt werden, 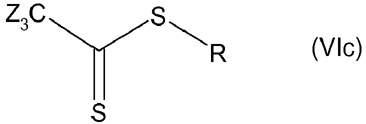
- Z: die zuvor für die allgemeine Formel (VI) genannten Bedeutungen besitzt,
- R: die zuvor für die allgemeine Formel (VI) für die Variante b) mit m=0 genannten Bedeutungen besitzt, allerdings mit der Einschränkung, dass R nach homolytischer Spaltung der S-R Bindung entweder ein sekundäres, tertiäres oder aromatisch stabilisiertes Radikal ausbildet.

Dieser besonders bevorzugte Regler der allgemeinen Formel (VIc) ergibt sich aus dem Regler der allgemeinen Formel (VI), wobei
- n und m: jeweils = 0 sind,
- t: gleich 1 ist,
- X: für C(Z)₂ steht,
- Z: die zuvor für die allgemeine Formel (VI) genannten Bedeutungen besitzt und
- R: die zuvor für die allgemeine Formel (VI) für die Variante b) mit m=0 genannten Bedeutungen besitzt, allerdings mit der Einschränkung, dass R nach homolytischer Spaltung der S-R Bindung entweder ein sekundäres, tertiäres oder aromatisch stabilisiertes Radikal ausbildet.

Besonders bevorzugt wird ein Regler der allgemeinen Formel (VIc) eingesetzt, bei dem
R mit der Maßgabe, dass R nach homolytischer Spaltung der S-R Bindung entweder ein sekundäres, tertiäres oder aromatisch stabilisiertes Radikal ausbildet,
- für einen linearen oder verzweigten, gesättigten oder ein- oder mehrfach ungesättigten, optional ein- oder mehrfach substituierten Alkylrest steht, bevorzugt einen entsprechenden C₃-C₂₀-Alkylrest, insbesondere für sec.-Butyl, tert.-Butyl, iso-Propyl, 1-Buten-3-yl, 2-Chloro-1-buten-2-yl, Propionsaeure-2-yl, Propionitril-2-yl, 2-Methylpropannitril-2-yl, 2-Methylpropionsaeure-2-yl oder 1H,1H,2-Keto-3-oxo-4H,4H,5H,5H-perfluorundecanyl, oder
- für einen gesättigten oder ungesättigten, optional ein- oder mehrfach substituierten Carbo-oder Heterocyclylrest steht, inbesondere für Cyclohexyl, Cumyl oder Cyclohexan-1-nitril-1-yl,
- für einen (Hetero)Arylrest steht, ganz besonders bevorzugt für einen C₆-C₂₄-(Hetero)Arylrest, inbesondere Phenyl, Pyridinyl oder Anthracenyl,
- für einen (Hetero)Arylalkylrest steht, ganz besonders bevorzugt für einen C₇-C₂₅-(Hetero)Arylalkylrest, insbesondere für Benzyl, Phenylethyl oder 1-Methyl-1-phenyleth-2-yl, oder
- für Thiocarboxy, Carbonyl, Carboxy, Oxo, Thioxo, Epoxy, sowie Salze der genannten Verbindungen steht.

### Asymmetrische Trithiocarbonate:

In einer weiteren bevorzugten Ausführungsform wird mindestens ein Regler der allgemeinen Formel (VId) eingesetzt, 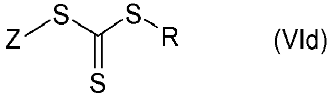 worin
- Z: die zuvor für die allgemeine Formel (VI) genannten Bedeutungen besitzt, allerdings mit der Einschränkung, dass Z nach homolytischer Spaltung der S-Z Bindung ein primäres Radikal ausbildet, und
- R: die gleichen Bedeutungen wie Z in der allgemeinen Formel (VI) besitzen kann, allerdings mit der Einschränkung, dass R nach homolytischer Spaltung der S-R Bindung entweder ein sekundäres, tertiäres oder aromatisch stabilisiertes Radikal ausbildet, und
mit der zusätzlichen Maßgabe, dass Z und R verschiedene Bedeutungen annehmen.

Dieser bevorzugte Regler der allgemeinen Formel (VId) ergibt sich aus dem Regler der allgemeinen Formel (VI) wobei
- n und m: jeweils = 0 sind,
- t: gleich 1 ist,
- X: für Schwefel steht,
- Z: die zuvor für die allgemeine Formel (VI) genannten Bedeutungen besitzt, allerdings mit der Einschränkung, dass Z nach homolytischer Spaltung der S-Z Bindung ein primäres Radikal ausbildet, und
- R: die gleichen Bedeutungen wie Z in der allgemeinen Formel (VI) besitzen kann, allerdings mit der Einschränkung, dass R nach homolytischer Spaltung der S-R Bindung entweder ein sekundäres, tertiäres oder aromatisch stabilisiertes Radikal ausbildet.

Bei diesen besonders bevorzugten Reglern der allgemeinen Formel (VId) handelt es sich somit um asymmetrische Trithiocarbonate.

Besonders bevorzugt wird ein Regler der oben genannten allgemeinen Formel (VId), worin
- **Z**: mit der Maßgabe, dass Z nach homolytischer Spaltung der S-Z Bindung ein primäres Radikal bildet, H, einen linearen oder verzweigten, gesättigten oder ein- oder mehrfach ungesättigten, optional ein- oder mehrfach substituierten Alkylrest, ganz besonders bevorzugt einen entsprechenden C₁-C₁₆ Alkylrest, insbesondere Methyl, Ethyl, n-Prop-1-yl, But-2-en-1-yl, n-Pent-1-yl, n-Hex-1-yl oder n-Dodecan-1-yl, Aralkyl, ganz besonders bevorzugt C₇-C₂₅-Aralkyl, insbesondere Benzyl, Amino, Amido, Carbamoyl, Hydroxyimino, Alkoxy, Aryloxy, F, Cl, Br, I, Hydroxy, Alkylthio, Arylthio, Carbonyl, Carboxy, Oxo, Thioxo, Cyanate, Thiocyanate, Isocyanate, Thioisocyanate, Isocyanide oder Salze der genannten Verbindungen bedeutet und
R mit der Maßgabe, dass R nach homolytischer Spaltung der S-R Bindung entweder ein sekundäres, tertiäres oder aromatisch stabilisiertes Radikal ausbildet,
- für einen linearen, verzweigten oder cyclischen, gesättigten oder ein- oder mehrfach ungesättigten, optional ein- oder mehrfach substituierten Alkylrest steht, bevorzugt einen entsprechenden C₃-C₂₀-Alkylrest, insbesondere für sec.-Butyl, tert.-Butyl, iso-Propyl, 1-Buten-3-yl, 2-Chloro-1-buten-2-yl, Propionsaeure-2-yl, Propionitril-2-yl, 2-Methylpropannitril-2-yl, 2-Methylpropionsaeure-2-yl oder 1H,1H,2-Keto-3-oxo-4H,4H,5H,5H-perfluorundecanyl, oder
- für einen gesättigten oder ungesättigten, optional ein- oder mehrfach substituierten Carbo-oder Heterocyclylrest steht, insbesondere für Cyclohexyl, Cumyl oder Cyclohexan-1-nitril-1-yl,
- für einen Arylrest oder Heteroarylrest steht, ganz besonders bevorzugt für einen C₆-C₂₄-Arylrest, insbesondere Phenyl, Pyridinyl oder Anthracenyl,
- für einen Aralkylrest steht, ganz besonders bevorzugt für Benzyl, Phenylethyl oder 1-Methyl-1-phenyleth-2-yl, oder
- für Thiocarboxy, Carbonyl, Carboxy, Oxo, Thioxo, Epoxy, sowie Salze der zuvor genannten Verbindungen steht.

### Dithioester:

In einer weiteren bevorzugten Ausführungsform wird mindestens ein Regler der allgemeinen Formel (VIe) eingesetzt, 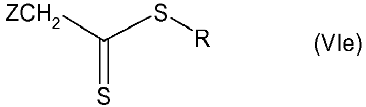 worin
- Z: alle für die allgemeine Formel (VI) genannten Bedeutungen besitzen kann und
- R: die gleichen Bedeutungen wie Z in der allgemeinen Formel (VI) besitzen kann, allerdings mit der Einschränkung, dass R nach homolytischer Spaltung der S-R Bindung entweder ein sekundäres, tertiäres oder aromatisch stabilisiertes Radikal ausbildet.

Dieser bevorzugte Regler der allgemeinen Formel (VIe) ergibt sich aus dem Regler der allgemeinen Formel (VI), wobei
n und m j eweils = 0 sind,
- t: gleich 1 ist,
- X: für CH₂ steht,
- Z: die zuvor für die allgemeine Formel (VI) genannten Bedeutungen besitzt und
- R: die gleichen Bedeutungen wie Z in der allgemeinen Formel (VI) besitzen kann, allerdings mit der Einschränkung, dass R nach homolytischer Spaltung der S-R Bindung entweder ein sekundäres, tertiäres oder aromatisch stabilisiertes Radikal ausbildet.

Besonders bevorzugt wird ein Regler der oben genannten allgemeinen Formel (VIe), worin
R mit der Maßgabe, dass R nach homolytischer Spaltung der S-R Bindung entweder ein sekundäres, tertiäres oder aromatisch stabilisiertes Radikal ausbildet,
- für einen linearen oder verzweigten, gesättigten oder ein- oder mehrfach ungesättigten, optional ein- oder mehrfach substituierten Alkylrest steht, bevorzugt einen entsprechenden C₃-C₂₀-Alkylrest, insbesondere für sec.-Butyl, tert.-Butyl, iso-Propyl, 1-Buten-3-yl, 2-Chloro-1-buten-2-yl, Propionsaeure-2-yl, Propionitril-2-yl, 2-Methylpropannitril-2-yl, 2-Methylpropionsaeure-2-yl oder 1H,1H,2-Keto-3-oxo-4H,4H,5H,5H-perfluorundecanyl, oder
- für einen gesättigten oder ungesättigten, optional ein- oder mehrfach substituierten Carbo-oder Heterocyclylrest steht, inbesondere für Cyclohexyl, Cumyl oder Cyclohexan-1-nitril-1-yl,
- für einen (Hetero)Arylrest steht, ganz besonders bevorzugt für einen C₆-C₂₄-(Hetero)Arylrest, inbesondere Phenyl, Pyridinyl oder Anthracenyl,
- für einen (Hetero)Arylalkylrest steht, ganz besonders bevorzugt für einen C₇-C₂₅-(Hetero)Arylalkylrest, insbesondere für Benzyl, Phenylethyl oder 1-Methyl-1-phenyleth-2-yl, oder
- für Thiocarboxy, Carbonyl, Carboxy, Oxo, Thioxo, Epoxy, sowie Salze der genannten Verbindungen steht.

Alle vorgenannten Regler sind nach Methoden, die dem Fachmann aus dem Stand der Technik geläufig sind, synthetisierbar. Synthesevorschriften und weitere Verweise für Herstellvorschriften können beispielsweise aus Polymer 49 (2008) 1079-1131 sowie allen in dieser Anmeldung als Stand der Technik bereits genannten Schutzrechten und Literaturstellen entnommen werden. Eine Reihe der Regler sind auch bereits käuflich erhältlich.

Besonders geeignet sind als Regler für das erfindungsgemäße Verfahren Dodecylpropansäuretrithiocarbonat (DoPAT), Dibenzoyltrithiocarbonat (DiBenT), Cumylphenyldithioacetat (CPDA), Cumyldithiobenzoat, Phenylethyldithiobenzoat, Cyanoisopropyldithiobenzoat, 2-Cyanoethyldithiobenzoat, 2-Cyanoprop-2-yl-dithiophenylacetat, 2-Cyanoprop-2-yl-dithiobenzoat, S-Thiobenzoyl-1H, 1H, 2-Keto-3 -oxa-4H,4H,5H,5H-perfluoroundecanthiol und S-Thiobenzoyl-1-phenyl-2-keto-3-oxa-4H,4H,5H,5H-perfluoro-undecanthiol.

Üblicherweise werden hier 1 bis 2000 mol% des Reglers bezogen auf 1 mol des Initiators eingesetzt. Bevorzugt werden 2 bis 1000 mol% des Reglers bezogen auf 1 mol des Initiators eingesetzt, besonders bevorzugt 4 bis 100 mol%.

Erfindungsgemäß bevorzugt wird der zu kuppelnde Nitrilkautschuk erhalten durch radikalische Polymerisation mindestens eines konjugierten Diens, mindestens eines α,β-ungesättigten Nitrils und gegebenenfalls eines oder mehrerer weiterer copolymerisierbarer Monomere, in Gegenwart mindestens eines organischen Lösungsmittels und mindestens eines Reglers, wobei als Regler mindestens eine Verbindung der allgemeinen Formel (I) 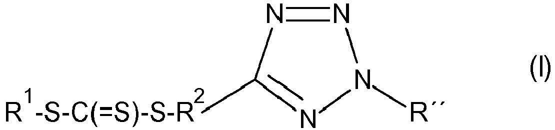 mit der Bedeutung
- R¹: Kohlenwasserstoffrest, beispielsweise C₅-C₂₀-Kohlenwasserstoffrest, der substituiert sein kann und nach homolytischer Spaltung der R¹-S-Bindung ein primäres Radikal ausbildet,
- R²: Kohlenwasserstoffrest, beispielsweise C₁₋C₁₂-Kohlenwasserstoffrest, der substituiert sein kann, eine oder mehrere Carboxylgruppen enthalten kann und nach homolytischer Spaltung der S-R²-Bindung ein sekundäres, tertiäres oder aromatisch stabilisiertes Radikal ausbildet,
- R": ein beliebiger Aromat, z. B. Phenyl, Benzyl, eingesetzt wird.

Für die Definition möglicher Substituenten, primärer radikaler, sekundärer, tertiärer oder aromatisch stabilisierter Radikale kann auf die vorstehenden Ausführungen verwiesen werden.

Bevorzugt ist in der Verbindung der allgemeinen Formel (I) R¹ ein C₈-C₁₅-Alkylrest, insbesondere C₁₀₋C₁₄-Alkylrest, insbesondere C₁₁-C₁₃-Alkylrest. Besonders bevorzugt handelt es sich dabei um lineare Alkylreste.

R² ist vorzugsweise ein Rest -CHR³-C(=O)OR⁴- mit R³ C₁₋C₃-Alkyl, besonders bevorzugt Methyl oder Ethyl, insbesondere Methyl, und R⁴ ist ein C₁₋C₆-Alkylenrest, der durch Carboxyl-und/oderArylgruppen unterbrochen sein kann. R" ist vorzugsweise Phenyl, Tolyl, Naphthyl oder Anthracenyl.

Diese erfindungsgemäße Ausführungsform weist den Vorteil auf, dass hohe Molekulargewichte unter Einsatz kleiner Initiatormengen zugänglich sind und eine sehr hohe Endgruppen-Treue erreicht wird, d. h. dass in den erhaltenen Polymer-Molekülen ein hoher Anteil an Tetrazol-Endgruppen vorliegt. Bezogen auf den Initiator werden die Regler der allgemeinen Formel (I) vorzugsweise in einer Menge von 5 bis 2000 mol-%, besonders bevorzugt 20 bis 1500 mol-%, insbesondere 500 bis 1500 mol-% eingesetzt. Dies bedeutet insbesondere einen 4- bis 14-fachen molaren Überschuss an RAFT-Regler der allgemeinen Formel (I). Die hohe Endgruppen-Treue bedeutet andererseits eine hohe Funktionalitätsdichte, so dass ein hoher Anteil an Tetrazol-Gruppen, bezogen auf die Polymerketten, vorliegt.

Die für die Umsetzung mit dem Regler der allgemeinen Formel (I) geeigneten Initiatoren und Monomere sowie Lösungsmittel werden nachfolgend aufgeführt. Diese beziehen sich auf die Herstellung der NBR mit dem Regler der allgemeinen Formel (VI).

### INITIATOREN:

Bei dem erfindungsgemäßen Verfahren handelt es sich um eine radikalische Polymerisation. Auf welche Weise diese initiiert wird, ist dabei nicht kritisch, insofern kommt eine Initiierung durch peroxidische Initiatoren, Azo-Initiatoren, Redox-Systeme oder auf photochemischem Weg in Frage. Unter diesen Initiatoren sind die Azo-Initiatoren bevorzugt.

Als Azo-Initiatoren können beispielsweise die folgenden Verbindungen eingesetzt werden: 2,2'-Azobis(isobutyronitril), 2,2'-Azobis(2-cyano-2-butan), Dimethyl-2,2'-azobisdimethyl-isobutyrat, 4,4'-Azobis(4-cyanopentansäure), 2-(t-Butylazo)-2-cyanopropan, 2,2'-Azobis[2-methyl-N-(1,1)-bis(hydroxymethyl)-2-hydroxyethyl] propionamid, 2,2'-Azobis[2-methyl-N-hydroxyethyl]-propionamid, 2,2'-Azobis(N,N-dimethylenisobutyr-amidine) dihydrochlorid, 2,2'-Azobis(2-amidinopropan)dihydrochlorid, 2,2'-Azobis(N,N'-dimethylenisobutyramin), 2,2'-Azobis(2-methyl-N-[1,1-bis(hydroxymethyl)-2-hydroxyethyl] propionamid), 2,2'-Azo-bis(2-methyl-N-[1,1-bis(hydroxymethyl)ethyl]propionamid), 2,2'-Azobis[2-methyl-N-(2-hydroxyethyl) propionamid], 2,2'-Azobis(isobutyramid)dihydrat, 2,2'-Azobis(2,2,4-trimethylpentan) 2,2'-Azobis(2-methylpropan), 1,1'-Azobis(cyclohexan-1-carbonitril), 2,2'-Azobis[N-(2-propenyl)-2-methylpropionamid], 1-[(1-Cyano-1-methylethyl)azo]formamid, 2,2'-Azobis(N-butyl-2-methylpropionamid), 2,2'-Azobis-(N-cyclohexyl-2-methylpropionamid) und 2,2'-Azobis(2,4,4-trimethylpentan).

Üblicherweise werden die Azo-Initiatoren in einer Menge 10⁻⁴ bis 10⁻¹ mol/1, bevorzugt in einer Menge von 10⁻⁴ bis 10⁻² mol/1 eingesetzt. Durch Abstimmung des Verhältnisses der eingesetzten Initiatormenge zur Menge des verwendeten Reglers gelingt es, sowohl die Reaktionskinetik als auch die Molekularstruktur (Molekulargewicht, Polydispersität) gezielt zu beeinflussen.

Als peroxidische Initiatoren können beispielsweise die folgenden Peroxo-Verbindungen, die eine -O-O-Einheit aufweisen, eingesetzt werden: Wasserstoffperoxid, Peroxodisulfate, Peroxodiphosphate, Hydroperoxide, Persäuren, Persäureester, Persäureanhydride und Peroxide mit zwei organischen Resten. Als Salze der Peroxodischwefelsäure und der Peroxodiphosphorsäure können Natrium-, Kalium- und Ammoniumsalze eingesetzt werden. Geeignete Hydroperoxide sind z.B. t-Butylhydroperoxid, Cumolhydroperoxid, Pinanhydroperoxid und p-Menthanhydroperoxid. Geeignete Peroxide mit zwei organischen Resten sind Dibenzoylperoxid, 2,4-Dichlorbenzoylperoxid, 2,5-Dimethylhexan-2,5-di-t.butylperoxid, Bis-(t-butylperoxy-isopropyl)benzol, t-Butylcumylperoxid, Di-t-butylperoxid, Dicumylperoxid, t-Butylperbenzoat, t-Butylperacetat, 2,5-Dimethylhexan-2,5-diperbenzoat, t-Butyl-per-3,5,5-trimethylhexanoat. Bevorzugt wird p-Menthanhydroperoxid, Cumolhydroperoxid, Pinanhydroperoxid oder 1,1-Di(tert.-butylperoxi)-3,3,5-trimethylcyclohexan eingesetzt.

In einer alternativen Ausführungsform werden Azo- oder peroxidische Initiatoren mit einer verlängerten Zerfallszeit eingesetzt. Hierbei hat es sich bewährt, den Azo-Initiator bzw. den peroxidischen Initiator so zu wählen, dass bei einer Temperatur von 70°C bis 200 °C, bevorzugt 80°C bis 175°C, besonders bevorzugt 85°C bis 160°C und insbesondere 90°C bis 150°C, die Halbwertszeit des jeweiligen Initiators im gewählten Lösungsmittel 10 Stunden oder mehr als 10 Stunden beträgt. Bevorzugt sind dabei Azo-Initiatoren, die bei einer Temperatur von 70°C bis 200 °C, bevorzugt 80°C bis 175°C, besonders bevorzugt 85°C bis 160°C und ganz besonders bevorzugt 90°C bis 150°C, eine Halbwertszeit von 10 Stunden oder mehr als 10 Stunden im gewählten Lösungsmittel besitzen. Besonders bevorzugt werden Azo-Initiatoren der folgenden Strukturformeln (Ini-1)- (Ini-6) eingesetzt:

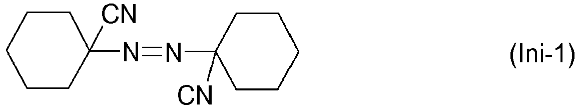

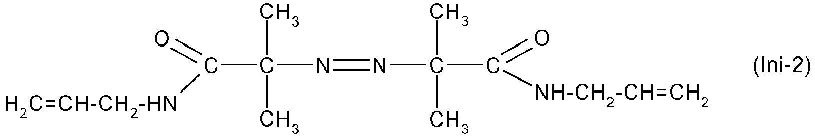

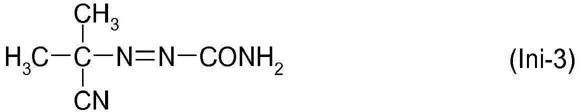

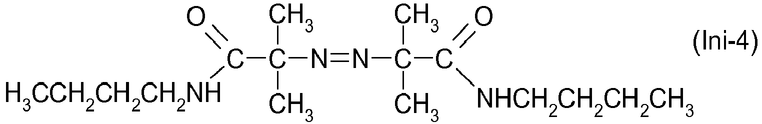

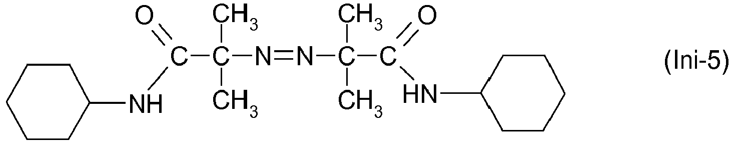

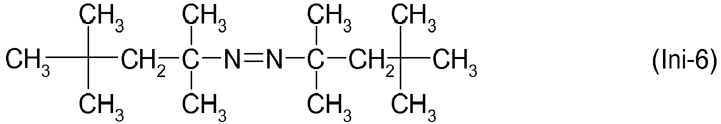

Ganz besonders bevorzugt ist der Einsatz der Initiatoren der Formel (Ini 1), (Ini-2) und (Ini-3). Die vorgenannten Azo-Initiatoren der Strukturformeln (Ini-1) - (Ini-6) sind käuflich erhältlich, beispielsweise von Wako Pure Chemical Industries, Ltd..

Der Begriff der Halbwertszeit ist dem Fachmann im Zusammenhang mit Initiatoren geläufig. Nur als Beispiel: Eine Halbwertszeit von 10 Stunden in einem Lösungsmittel bei einer bestimmten Temperatur bedeutet konkret, dass sich unter diesen Bedingungen nach 10 Stunden die Hälfte des Initiators zersetzt hat.

Bei Einsatz der vorgenannten bevorzugten Initiatoren mit einer höheren Zerfallstemperatur, insbesondere der genannten Azoinitiatoren lassen sich Nitrilkautschuke mit vergleichsweise höheren mittleren Molekulargewichten Mw (Gewichtsmittel des Molekulargewichts) und Mn (Zahlenmittel des Molekulargewichts) synthetisieren, die sich gleichzeitig weiterhin durch eine hohe Linearität auszeichnen. Dies kommt zum Ausdruck durch entsprechend niedrige Werte der Mooney-Relaxation, gemessen durch ISO 289 Teil 1 & 2 oder alternativ nach ASTM D1646.

Als Redox-Systeme können die folgenden Systeme aus einem Oxidationsmittel und einem Reduktionsmittel eingesetzt werden. Die Wahl geeigneter Mengen an Oxidations- und Reduktionsmittel ist dem Fachmann hinreichend geläufig.

Bei der Verwendung von Redoxsystemen verwendet man häufig zusätzlich Salze von Übergangsmetallverbindungen wie Eisen, Cobalt oder Nickel in Kombination mit geeigneten Komplexbildnern wie Natrium-Ethylendiamintetraacetat, Natrium-Nitrilotriacetat sowie Trinatriumphosphat oder Tetrakaliumdiphosphat.

Als Oxidationsmittel können dabei beispielsweise alle Peroxoverbindungen, die zuvor für die peroxidischen Initiatoren genannt wurden, eingesetzt werden.

Als Reduktionsmittel können im erfindungsgemäßen Verfahren beispielsweise die folgenden eingesetzt werden: Natriumformaldehydsulfoxylat, Natriumbenzaldehydsulfoxylat, reduzierende Zucker, Ascorbinsäure, Sulfenate, Sulfinate, Sulfoxylate, Dithionit, Sulfit, Metabisulfit, Disulfit, Zucker, Harnstoff, Thioharnstoff, Xanthogenate, Thioxanthogenate, Hydraziniumsalzen, Amine und Aminderivate wie Anilin, Dimethylanilin, Monoethanolamin, Diethanolamin oder Triethanolamin. Bevorzugt wird Natriumformaldehydsulfoxylat eingesetzt.

Die Initiierung der radikalischen Polymerisation kann auch photochemisch wie im Folgenden beschrieben erfolgen: Hierzu wird dem Reaktionsgemisch ein Photoinitiator zugesetzt, welcher durch Bestrahlung mittels Licht geeigneter Wellenlänge angeregt wird und eine radikalische Polymerisation initiiert. Es ist hierbei anzumerken, dass für die optimale Initiierung der radikalischen Polymerisation die Bestrahlungsdauer von der Leistung des Strahlers, von dem Abstand zwischen dem Strahler und dem Reaktionsgefäß sowie von der Bestrahlungsfläche abhängig ist. Es ist jedoch für den Fachmann ohne weiteres durch verschiedene Testreihen möglich, die optimale Bestrahlungsdauer herauszufinden. Auch die Wahl der geeigneten Initiatormenge ist für den Fachmann ohne Probleme möglich und dient zur Beeinflussung des Zeit-Umsatz-Verhaltens der Polymerisation.

Als photochemische-Initiatoren können beispielsweise die Folgenden eingesetzt werden: Benzophenon, 2-Methylbenzophenon, 3,4-Dimethylbenzophenon, 3-Methylbenzophenon, 4,4'-Bis(diethylamino)benzophenon, 4,4'-Di-hydroxybenzophenon, 4,4'-Bis[2-(1-propenyl)phenoxy]benzophenon, 4-(Diethylamino)benzophenon, 4-(Dimethylamino)benzophenon, 4-Benzoylbiphenyl, 4-Hydroxybenzophenon, 4-Methylbenzophenon, Benzophenon-3,3',4,4'-tetracarboxyldianhydrid, 4,4'-Bis(dimethylamino)benzophenon, Acetophenon, 1-Hydroxycyclohexylphenylketon, 2,2-Diethoxyacetophenon, 2,2-Dimethoxy-2-phenylacetophenon, 2-Benzyl-2-(dimethylamino)-4'-morpholino-butyrophenon, 2-Hydroxy-2-methylpropiophenon, 2-Hydroxy-4'-(2-hydroxyethoxy)-2-methylpropiophenon, 3'-Hydroxyacetophenon, 4'-Ethoxyacetophenon, 4'-Hydroxyacetophenon, 4'-Phenoxyacetophenon, 4'-tert-Butyl-2',6'-dimethylacetophenon, 2-Methyl-4'-(methylthio)-2-morpholinopropiophenon, Diphenyl-(2,4,6-trimethylbenzoyl)-phosphinoxid, Phenylbis(2,4,6-trimethylbenzoyl)phosphinoxid, Methylbenzoylformat, Benzoin, 4,4'-Dimethoxybenzoin, Benzoinmethylether, Benzoinethylether, Benzoinisopropylether, Benzoinisobutylether, 4,4'-Dimethylbenzil, Hexachlorocyclopentadiene oder Kombinationen daraus.

### Lösungsmittel

Das erfindungsgemäße Verfahren wird vorzugsweise in einem organischen Lösungs- oder Verdünnungsmittel durchgeführt. Dies trifft sowohl für den Polymerisationsschritt als auch die eigentliche Kupplungsreaktion zu. Große Mengen Wasser, wie im Fall der Emulsionspolymerisation sind somit nicht im Reaktionssystem vorhanden. Geringere Mengen Wasser in der Größenordnung von bis zu 5 Gew.%, bevorzugt bis 1 Gew.% (bezogen auf die Menge des organischen Lösungsmittels) können durchaus im Reaktionssystem zugegen sein. Entscheidend ist, dass die Mengen anwesenden Wassers so gering gehalten werden muss, dass es nicht zu einer Ausfällung des sich bildenden NBR-Polymers kommt. Es sei an dieser Stelle klar gestellt, dass es sich bei dem erfindungsgemäßen Verfahren nicht um eine Emulsionspolymerisation handelt.

Als organische Lösungsmittel geeignet sind beispielsweise Aceton, Acetonitril, Dimethylacetamid, Monochlorbenzol, Dichlormethan, Toluol, Ethylacetat, 1,4-Dioxan, t-Butanol, Isobutyronitril, 3-Propanon, Dimethylcarbonat, 4-Methylbutan-2-on und Methylethylketon. Bevorzugt sind polare Lösungsmittel, welche einen Hildebrand'schen Lösungsparameter δ (δ = ((ΔHᵥ-RT)/Vₘ)^{½} [(MPa)^{½}]) (Vₘ = molares Volumen; ΔHᵥ = Verdampfungsenthalpie; R = ideale Gaskonstante)) in einem Bereich zwischen 15,5 und 26 (MPa) ^{½} besitzen.

Ausschlaggebend für die Eignung eines Lösungsmittels ist, dass der hergestellte Nitrilkautschuk während der Polymerisation, der sich anschließenden Aufarbeitung und während des Kupplungsschrittes vollständig in Lösung bleibt. Nicht eingesetzt werden können Lösungsmittel, die als Transferreagenzien in die Reaktion eingreifen, z.B. Tetrachlorkohlenstoff, Thiole und weitere dem Fachmann als solche bekannte Solventien dieser Art, sowie Lösungsmittel, die stark UV-absorbierend wirken.

Ebenfalls möglich ist es, ein Gemisch von zwei oder mehr organischen Lösungsmitteln einzusetzen. Möglich ist es auch, Lösungsmittel einzusetzen, die den og. Anforderungen genügen und einen Siedepunkt besitzen, der unterhalb desjenigen von Acrylnitril liegt, wie z.B. Methyl-tert.butylether (MTBE).

### Temperatur:

Das erfindungsgemäße Verfahren der Polymerisation wird üblicher Weise bei einer Temperatur in einem Bereich von 60°C bis 150°C durchgeführt, bevorzugt in einem Bereich von 70°C bis 130°C, besonders bevorzugt in einem Bereich von 80°C bis 120°C und insbesondere in einem Bereich von 90°C bis 110°C. Wird die Temperatur noch tiefer gewählt, ist die Polymerisation entsprechend verlangsamt. Bei noch deutlich höheren Temperaturen ist es nicht ausgeschlossen, dass der eingesetzte Initiator zu schnell zerfällt bzw. das RAFT-Agens zersetzt wird. Insbesondere beim Einsatz peroxidischer Initiatoren ist es nicht ausgeschlossen, dass es u.U. zur Oxidation des Reglers kommt.

Das erfindungsgemäße Verfahren der Kupplung kann allgemein in breiten Konzentrationsbereichen und bei beliebiger geeigneter Temperatur erfolgen. Vorzugsweise wird die Kupplung bei einer Temperatur im Bereich von 0 bis 200 °C, besonders bevorzugt 20 bis 140 °C, insbesondere etwa Raumtemperatur (22-26°C) durchgeführt. Dabei kann die Kupplung in der Regel an Luft erfolgen.

### Umsetzung:

Im Fall der Initiierung durch Peroxoverbindungen oder Azo-Initiatoren erfolgt die Durchführung des erfindungsgemäßen Verfahrens üblicherweise so, dass das α,β-ungesättigte Nitril und die optional eingesetzten weiteren copolymerisierbaren Monomere, das Lösungsmittel, der Initiator sowie der/die Regler in einem Reaktionsgefäß vorgelegt werden und anschließend das oder die konjugierte Diene zudosiert wird/werden. Die Polymerisation wird im Anschluss durch Temperaturerhöhung gestartet.

Im Falle einer Initiierung mittels eines Redox-Systems wird typischerweise das Oxidationsmittel zusammen mit einem der Monomeren in das Reaktionsgefäß dosiert. Die Polymerisation wird im Anschluss durch Zusatz des Reduktionsmittels gestartet.

Um spezielle Verhältnisse der jeweiligen Monomeren im Co-/Terpolymer zu erhalten, ist es sinnvoll und dem Fachmann durchweg geläufig, entsprechende Modifikationen bezüglich der Dosierung vorzunehmen (z.B. durch Nachdosieren des jeweiligen Monomers, von Initiator-Mengen, ReglerMengen oder Lösungsmittel). Diese Nachdosierungen können dabei entweder kontinuierlich oder aber auch in einzelnen Portionen diskontinuierlich erfolgen. Auch die Nachdosierung der Monomere oder aber die Nachdosierung von Initiator kann entweder kontinuierlich oder aber auch in einzelnen Portionen diskontinuierlich erfolgen.

Zur Einstellung eines geeigneten Molekulargewichts sowie zwecks Erreichens des gewünschten Umsatzes hat es sich in einer Ausführungsform des erfindungsgemäßen Verfahrens bewährt, den Initiator im Verlauf der Polymerisationsreaktion ein- oder mehrmals nachzudosieren.

Das konjugierte Dien im Nitrilkautschuk kann von jeder Natur sein. Bevorzugt werden (C₄-C₆) konjugierte Diene eingesetzt. Besonders bevorzugt sind 1,2-Butadien, 1,3-Butadien, Isopren, 2,3-Dimethylbutadien, Piperylen oder Gemische daraus. Insbesondere bevorzugt sind 1,3-Butadien und Isopren oder Gemische daraus. Ganz besonders bevorzugt ist 1,3-Butadien.

Als α,β-ungesättigtes Nitril kann jedes bekannte α,β-ungesättigte Nitril eingesetzt werden, bevorzugt sind (C₃-C₅)-α,β-ungesättigte Nitrile wie Acrylnitril, Methacrylnitril, Ethacrylnitril oder Mischungen davon. Besonders bevorzugt ist Acrylnitril.

Ein besonders bevorzugter Nitrilkautschuk ist ein Copolymer aus Acrylnitril und 1,3-Butadien.

Als weitere copolymerisierbare Termonomere können beispielsweise aromatische Vinylmonomere, bevorzugt Styrol, α-Methylstyrol und Vinylpyridin, fluorhaltige Vinylmonomere, bevorzugt Fluorethylvinylether, Fluorpropylvinylether, o-Fluormethylstyrol, Vinylpentafluorbenzoat, Difluoroethylen und Tetrafluoroethylen, oder auch copolymerisierbare Antiageing Monomere, bevorzugt N-(4-anilinophenyl) acrylamid, N-(4-anilinophenyl) methacrylamid, N-(4-anilinphenyl) cinnamide, N-(4-anilinophenyl) crotonamid, N-phenyl-4-(3-vinylbenzyloxy) anilin und N-phenyl-4-(4-vinylbenzyloxy) anilin eingesetzt werden sowie nicht-konjugierte Diene, wie 4-Cyanocyclohexen und 4-Vinylcyclohexen, oder auch Alkine, wie 1- oder 2-Butin.

Alternativ können als weitere copolymerisierbare Termonomere carboxygruppenhaltige, copolymerisierbare Termonomere eingesetzt werden, beispielsweise α,β-ungesättigte Monocarbonsäuren, deren Ester, α,β-ungesättigte Dicarbonsäuren, deren Mono- oder -Diester oder um deren entsprechenden Anhydride oder Amide.

Als α,β-ungesättigte Monocarbonsäuren können bevorzugt Acrylsäure und Methacrylsäure eingesetzt werden.

Einsetzbar sind auch Ester der α,β-ungesättigten Monocarbonsäuren, bevorzugt deren Alkylester und Alkoxyalkylester. Bevorzugt sind die Alkylester, insbesondere C₁-C₁₈ Alkylester der α,β-ungesättigten Monocarbonsäuren. Besonders bevorzugt sind Alkylester, insbesondere C₁-C₁₈ Alkylester der Acrylsäure oder der Methacrylsäure, insbesondere Methylacrylat, Ethylacrylat, Propylacrylat, n-Butylacrylat, tert.-Butylacrylat, 2-Ethylhexylacrylat, n-Dodecylacrylat, Methylmethacrylat, Ethylmethacrylat, Butylmethacrylat und 2-Ethylhexyl-methacrylat. Bevorzugt sind auch Alkoxyalkylester der α,β-ungesättigten Monocarbonsäuren, besonders bevorzugt Alkoxyalkylester der Acrylsäure oder der Methacrylsäure, insbesondere C₂-C₁₂-Alkoxyalkylester der Acrylsäure oder der Methacrylsäure, ganz besonders bevorzugt Methoxymethylacrylat, Methoxyethyl(meth)acrylat, Ethoxyethyl(meth)acrylat und Methoxymethyl-(meth)acrylat. Einsetzbar sind auch Mischungen von Alkylestern, wie z.B. den vorgenannten, mit Alkoxyalkylestern, z.B. in Form der vorgenannten. Einsetzbar sind auch Cyanoalkylacrylate and Cyanoalkylmethacrylate, in denen die C-Atom-Zahl der Cyanoalkylgruppe 2-12 beträgt, vorzugsweise α-Cyanoethylacrylat, β-Cyanoethylacrylat und Cyanobutylmethacrylat. Einsetzbar sind auch Hydroxyalkylacrylate and Hydroxyalkylmethacrylate, in denen die C-Atom-Zahl der Hydroxyalkylgruppen 1-12 beträgt, vorzugsweise 2-Hydroxyethylacrylat, 2-Hydroxyethylmethacrylat und 3-Hydroxypropylacrylat; Einseztbar sind auch Fluor-substituierte Benzylgruppenhaltige Acrylate oder Methacrylate, vorzugsweise Fluorobenzylacrylate, und Fluorobenzylmethacrylat. Einsetzbar sind auch Fluoroalkylgruppen haltige Acrylate und -Methacrylate, vorzugsweise Trifluoroethylacrylat und Tetrafluoropropylmethacrylat. Einsetzbar sind auch Aminogruppenhaltige α,β-ungesättigte Carbonsäureester wie Dimethylaminomethylacrylat und Diethylaminoethylacrylat.

Als weitere copolymerisierbare Monomere können ferner α,β-ungesättigte Dicarbonsäuren, bevorzugt Maleinsäure, Fumarsäure, Crotonsäure, Itaconsäure, Citraconsäure und Mesaconsäure, eingesetzt werden.

Eingesetzt werden können ferner α,β-ungesättigte Dicarbonsäureanhydride, bevorzugt Maleinsäureanhydrid, Itaconsäureanhydrid, Citraconsäureanhydrid und Mesaconsäureanhydrid. Eingesetzt werden können ferner Mono- oder Diester von α,β-ungesättigten Dicarbonsäuren.

Bei diesen α,β-ungesättigten Dicarbonsäuremono- oder diestern kann es sich beispielsweise handeln um Alkyl-, bevorzugt C₁-C₁₀-Alkyl, insbesondere Ethyl-, n-Propyl-, iso-Propyl, n-Butyl-, tert.-Butyl, n-Pentyl- oder n-Hexyl-, Alkoxyalkyl-, bevorzugt C₂-C₁₂ Alkoxyalkyl-, besonders bevorzugt C₃-C₈- Alkoxyalkyl, Hydroxyalkyl, bevorzugt C₁-C₁₂ Hydroxyalkyl-, besonders bevorzugt C₂-C₈- Hydroxyalkyl, Cycloalkyl-, bevorzugt C₅-C₁₂-Cycloalkyl-, besonders bevorzugt C₆-C₁₂-Cycloalkyl, Alkylcycloalkyl-, bevorzugt C₆-C₁₂-Alkylcycloalkyl-, besonders bevorzugt C₇-C₁₀-Alkylcycloalkyl, Aryl-, bevorzugt C₆-C₁₄-Aryl-Mono- oder -Diester, wobei es sich im Fall der Diester jeweils auch um gemischte Ester handeln kann.

Besonders bevorzugte Alkylester von α,β-ungesättigten Monocarbonsäuren sind Methyl(meth)acrylat, Ethyl(meth)acrylat, Propyl(meth)acrylat, n-Butyl(meth)acrylat, t-Butyl(meth)acrylat, Hexyl(meth)acrylat, 2-Ethylhexyl(meth)acrylat, Octyl(meth)acrylat, 2-Propylheptylacrylat und Lauryl(meth)acrylat. Insbesondere wird n-Butylacrylat eingesetzt.

Besonders bevorzugte Alkoxyalkylester der α,β-ungesättigten Monocarbonsäuren sind Methoxyethyl(meth)acrylat, Ethoxyethyl(meth)acrylat und Methoxymethyl(meth)acrylat. Insbesondere wird Methoxyethylacrylat eingesetzt.

Besonders bevorzugte Hydroxyalkylester der α,β-ungesättigten Monocarbonsäuren sind Hydroxyethyl(meth)acrylat, Hydroxypropyl-(meth)acrylat und Hydroxybutyl(meth)acrylat.

Als sonstige Ester der α,β-ungesättigten Monocarbonsäuren werden ferner beispielsweise Polyethylenglykol(meth)acrylat, Polypropylenglykol(meth)acrylat, Glycidyl(meth)acrylat, Epoxy(meth)acrylat, N-(2-Hydroxyethyl)acrylamide, N-(2-Hydroxymethyl)acrylamide und Urethan(meth)acrylat eingesetzt.

Beispiele von α,β-ungesättigten Dicarbonsäuremonoestern umfassen
- Maleinsäuremonoalkylester, bevorzugt Monomethylmaleat, Monoethylmaleat, Monopropyl maleat und Mono-n-butylmaleat;
- Maleinsäuremonocycloalkylester, bevorzugt Monocyclopentylmaleat, Monocyclohexyl maleat und Monocycloheptylmaleat;
- Maleinsäuremonoalkylcycloalkylester, bevorzugt Monomethylcyclopentylmaleat und Monoethylcyclohexylmaleat;
- Maleinsäuremonoarylester, bevorzugt Monophenylmaleat;
- Maleinsäuremonobenzylester, bevorzugt Monobenzylmaleat;
- Fumarsäuremonoalkylester, bevorzugt Monomethylfumarat, Monoethylfumarat, Monopropyl fumarat und Mono-n-butyl fumarat;
- Fumarsäuremonocycloalkylester, bevorzugt Monocyclopentylfumarat, Monocyclohexyl fumarat und Monocycloheptylfumarat;
- Fumarsäuremonoalkylcycloalkylester, bevorzugt Monomethylcyclopentylfumarat und Monoethylcyclohexylfumarat;
- Fumarsäuremonoarylester, bevorzugt Monophenylfumarat;
- Fumarsäuremonobenzylester, bevorzugt Monobenzylfumarat;
- Citraconsäuremonoalkylester, bevorzugt Monomethylcitraconat, Monoethylcitraconat, Monopropylcitraconat und Mono-n-butyl citraconat;
- Citraconsäuremonocycloalkylester, bevorzugt Monocyclopentylcitraconat, Monocyclohexyl citraconat und Monocycloheptylcitraconat;
- Citraconsäuremonoalkylcycloalkylester, bevorzugt Monomethylcyclopentylcitraconat und Monoethylcyclohexylcitraconat;
- Citraconsäuremonoarylester, bevorzugt Monophenylcitraconat;
- Citraconsäuremonobenzylester, bevorzgut Monobenzylcitraconat;
- Itaconsäuremonoalkylester, bevorzugt Monomethylitaconat, Monoethylitaconat, Monopropyl itaconat und Mono-n-butyl itaconat;
- Itaconsäuremonocycloalkylester, bevorzugt Monocyclopentylitaconat, Monocyclohexyl itaconat und Monocycloheptylitaconat;
- Itaconsäuremonoalkylcycloalkylester, bevorzugt Monomethylcyclopentylitaconat und Monoethylcyclohexylitaconat;
- Itaconsäuremonoarylester, bevorzugt Monophenylitaconat;
- Itaconsäuremonobenzylester, bevorzugt Monobenzylitaconat.
- Mesaconsäuremonoalkylester, bevorzugt Mesaconsäuremonoethylester;

Als α,β-ungesättigte Dicarbonsäurediester können die analogen Diester basierend auf den zuvor genannten Monoestergruppen eingesetzt werden, wobei es sich bei den Estergruppen auch um chemisch verschiedene handeln kann.

Es ist ferner möglich, als weitere copolymerisierbare Monomere radikalisch polymerisierbare Verbindungen einzusetzen, die pro Molekül zwei oder mehr olefinische Doppelbindungen enthalten. Beispiele solcher zwei- oder mehrfach ungesättigter Verbindungen sind zwei- oder mehrfach ungesättigte Acrylate, Methacrylate oder Itaconate von Polyolen wie z.B. 1,6-Hexandioldiacrylat (HDODA), 1,6-Hexandioldimethacrylat, Ethylenglykoldiacrylat, Ethylenglykoldimethacrylat (EGDMA), Diethylenglykoldimethacrylat, Triethylenglykoldiacrylat, Butandiol-1,4-diacrylat, Propandiol-1,2-diacrylat, Butandiol-1,3-dimethacrylat, Neopentylglykoldiacrylat, Trimethylolpropandiacrylat, Trimethylolpropandimethacrylat, Trimethylolethandiacrylat, Trimethylolethandimethacrylat, Trimethylolpropantriacrylat, Trimethylolpropantrimethacrylat (TMPTMA), Glycerindi- und -triacrylat, Pentaerythritdi-, -tri-und tetraacrylat oder -methacrylat, Dipentaerythrittetra, -penta- und -hexaacrylat oder -methacrylat oder -itaconat, Sorbittetraacrylat, Sorbithexamethacrylat, Diacrylate oder Dimethacrylate von 1,4-Cyclohexandiol, 1,4-Dimethylolcyclohexan, 2,2-Bis(4-hydroxyphenyl)propan, von Polyethylenglykolen oder von Oligoestern oder Oligourethanen mit endständigen Hydroxylgruppen. Als mehrfach ungesättigte Monomere können auch Acrylamide verwendet werden wie z.B. Methylenbisacrylamid, Hexamethylen-1,6-bisacrylamid, Diethylentriamin-tris-methacrylamid, Bis(methacrylamido-propoxy)ethan oder 2-Acrylamido-ethylacrylat. Beispiele für mehrfach ungesättigte Vinyl- und Allylverbindungen sind Divinylbenzol, Ethylenglykoldivinylether, Diallylphthalat, Allylmethacrylat, Diallylmaleat, Triallylisocyanurat oder Triallylphosphat.

Bei Einsatz derartiger Termonomere gelingt es vorteilhafterweise, die Polymerisation bis zu hohen Umsätzen zu führen und dabei Nitrilkautschuke herzustellen, die ein vergleichsweise höheres mittleres Molekulargewicht Mw (Gewichtsmittel) bzw. Mn (Zahlenmittel) aufweisen, jedoch trotzdem gelfrei sind.

Die Anteile an konjugiertem Dien und α,β-ungesättigtem Nitril in den erhaltenen NBR-Polymeren können in weiten Bereichen schwanken. Der Anteil des oder der Summe der konjugierten Diene liegt üblicherweise im Bereich von 40 bis 90 Gew.-%, bevorzugt im Bereich von 50 bis 85 Gew. %, bezogen auf das Gesamtpolymer. Der Anteil des oder der Summe der α,β-ungesättigten Nitrile liegt üblicherweise bei 10 bis 60 Gew.-%, bevorzugt bei 15 bis 50 Gew.-%, bezogen auf das Gesamtpolymer. Die Anteile der Monomere summieren sich jeweils zu 100 Gew.-% auf. Die zusätzlichen Monomere können je nach Art des/der Termonomere in Mengen von 0 bis 40 Gew.%, bezogen auf das Gesamtpolymer, vorliegen. In diesem Fall werden entsprechende Anteile des oder der konjugierten Diene und/oder des oder der α,β-ungesättigten Nitrile durch die Anteile der zusätzlichen Monomere ersetzt, wobei sich die Anteile aller Monomere jeweils zu 100 Gew.-% aufsummieren.

Soweit es sich bei den Termonomeren um solche Monomere handelt, die tertiäre Radikale bilden (z.B. Methacrylsäure), hat es sich bewährt, diese in Mengen von 0 bis 10 Gew.% einzusetzen.

Es ist anzumerken, dass die zuvor genannte Begrenzung der zusätzlichen Monomere auf max. 40% nur für die Konstellation gilt, dass die Gesamtmenge an Monomeren dem Polymerisationsansatz zu Beginn bzw. während der Reaktion zudosiert werden (also zur Erzeugung statistischer Terpolymersysteme). Natürlich ist es möglich, einen erfindungsgemäß hergestellten optional hydrierten Nitrilkautschuk aufgrund der Tatsache, dass er in der Polymerhauptkette und/oder den Endgruppen Fragmente des oder der eingesetzten Regler aufweist, als Makro-Regler einzusetzen und durch Umsatz mit geeigneten Monomere in beliebiger Menge z.B. zur Generierung von Block-Systemen einzusetzen.

Die Glastemperaturen der erfindungsgemäßen optional hydrierten Nitrilkautschuke liegen im Bereich von -70°C bis +20°C, vorzugsweise im Bereich -60°C bis 10°C.

Aufgrund des lebenden Charakters der Polymerisation über das erfindungsgemäße Verfahren ist es möglich, Nitrilkautschuke mit einer engen Molekulargewichtsverteilung zu erhalten. Hergestellt werden können Nitrilkautschuke mit einem Polydispersitätsindex im Bereich von 1,0 bis 2,9, bevorzugt im Bereich von 1,1 bis 2,8, besonders bevorzugt im Bereich von 1,15 bis 2,7 und insbesondere im Bereich von 1,2 bis 2,6.

Aufgrund des lebenden Charakters der Polymerisation über das erfindungsgemäße Verfahren ist es sogar möglich, Nitrilkautschuke mit einer extrem engen Molekulargewichtsverteilung zu erhalten. Hergestellt werden können Nitrilkautschuke mit einem Polydispersitätsindex im Bereich von 1,1 bis 2,5, bevorzugt in einem Bereich von 1,3 bis 2,4, besonders bevorzugt in einem Bereich von 1,4 bis 2,2, insbesondere in einem Bereich von 1,5 bis 2,0, ganz besonders bevorzugt in einem Bereich von 1,3 bis kleiner 2.

Das erfindungsgemäße Verfahren erlaubt durch Steuerung der Reglerkonzentration eine sehr genaue Einstellung des gewünschten Molekulargewichts und darüber hinaus durch Einsatz der Regler auch den Aufbau gezielter Polymer-Architekturen (z.B. Herstellung von Blöcken, Graften auf Polymerbackbones, Oberflächenanbindung, der Einsatz von Termonomeren mit mehr als einer C=C Doppelbindung, sowie weitere dem Fachmann bekannten Polymermodifikationen) sowie gezielter Molekulargewichtsverteilungen von extrem engen bis hin zu breiten Verteilungen, von mono- über bibis hin zu multimodalen Verteilungen. Die über diese Methoden gezielt aufgebauten Nitrilkautschuke können einen Polydispersitätsindex PDI = Mw/Mn, wobei Mw das Gewichtsmittel und Mn das Zahlenmittel des Molekulargewichts darstellt, im Bereich von 1,1 bis 8,0, bevorzugt im Bereich von 1,15 bis 7,0, besonders bevorzugt im Bereich von 1,2 bis 6,0 und insbesondere im Bereich von 1,3 bis 5,0 besitzen.

### Hydrierung:

Ein weiterer Gegenstand der vorliegenden Erfindung besteht in der Bereitstellung von hydrierten Nitrilkautschuken, indem sich an den ersten Polymerisationsschritt a) bei Verwendung des Reglers der allgemeinen Formel (VI), unmittelbar, d. h. vor Anbindung der Tetrazol-Gruppe, b) bei Verwendung des Reglers der allgemeinen Formel (I), unmittelbar d. h. einschliessslich der Tetrazol-Gruppe, die Hydrierung c) anschließt, wobei keine vorhergehende Isolierung des Nitrilkautschuks nötig ist. Die Hydrierung kann unmittelbar im Anschluss an die Polymerisation, sofern gewünscht sogar im gleichen Reaktor durchgeführt werden. Dies führt zu einer substantiellen Vereinfachung und damit zu wirtschaftlichen Vorteilen bei der Herstellung des HNBR.

Die Hydrierung kann unter Einsatz homogener oder heterogener Hydrierkatalysatoren wie in WO 2011/032832 beschrieben, durchgeführt werden.

Bei den zu kuppelnden Nitrilkautschuken, die unter Verwendung der Regler der allgemeinen Formel (I) erhalten werden, wird eine Hydrierung erst nach der Kupplung durchgeführt. Die entsprechende Hydrierung kann ebenfalls wie vorstehend angegeben durchgeführt werden.

Sowohl die erfindungsgemäßen gekuppelten Nitrilkautschuke als auch die hydrierten gekuppelten Nitrilkautschuke zeichnen sich gegenüber den optional hydrierten Nitrilkautschuken, bei denen der Nitrilkautschuk durch Emulsionspolymerisation erhalten wird, dadurch aus, dass sie vollständig emulgatorfrei sind und auch keine Salze enthalten, wie sie üblicherweise zur Koagulation der Latices nach der Emulsionspolymerisation zwecks Ausfällung des Nitrilkautschukes eingesetzt werden.

Gegenstand der vorliegenden Erfindung sind ferner vulkanisierbare Mischungen enthaltend den gekuppelten, optional hydrierten Nitrilkautschuk und mindestens einen Vernetzer. In einer bevorzugten Ausführungsform handelt es sich um vulkanisierbare Mischungen, die zusätzlich mindestens einen Füllstoff enthalten.

Optional können derartige vulkanisierbare Mischungen auch noch ein oder mehrere dem Fachmann für Kautschuke geläufige Additive enthalten. Diese umfassen Alterungsschutzmittel, Reversionsschutzmittel, Lichtschutzmittel, Ozonschutzmittel, Verarbeitungshilfsmittel, Weichmacher, Mineralöle, Tackifier, Treibmittel, Farbstoffe, Pigmente, Wachse, Harze, Streckmittel, organische Säuren, Vulkanisationsverzögerer, Metalloxide, sowie weitere Füllstoffaktivatoren, wie beispielsweise Triethanolamin, Trimethylolpropan, Polyethylenglykol, Hexantriol, aliphatische Trialkoxysilane oder anderen Additive, die in der Gummündustrie bekannt sind (Ullmann's Encyclopedia of Industrial Chemistry, VCH Verlagsgesellschaft mbH, D-69451 Weinheim, 1993, vol A 23 "Chemicals and Additives", S. 366-417).

Als Vernetzer kommen beispielsweise peroxidische Vernetzer in Frage wie Bis(2,4-dichlorbenzyl)peroxid, Dibenzoylperoxid, Bis(4-chlorbenzoyl)peroxid, 1,1-Bis-(t-butylperoxy)-3,3,5-trimethylcyclohexan, tert-Butylperbenzoat, 2,2 Bis(t-butylperoxy) buten, 4,4-di-tert.Butyl peroxynonylvalerat, Dicumylperoxid, 2,5-Dimethyl-2,5-di(t-butylperoxy)-hexan, tert-Butylcumylperoxid, 1,3-Bis(t-butylperoxy isopropyl)-benzol, Di-t-butylperoxid und 2,5-Dimethyl-2,5-di(t-butylperoxy)-hexyn-3.

Es kann vorteilhaft sein, neben diesen peroxidischen Vernetzern noch weitere Zusätze zu verwenden, mit deren Hilfe die Vernetzungsausbeute erhöht werden kann: Hierfür sind beispielsweise Triallylisocyanurat, Triallylcyanurat, Trimethylolpropan-tri(meth)acrylat, Triallyltrimellithat, Ethylenglycoldimethacrylat, Butandioldimethacrylat, Trimethylolpropantrimethacrylat, Zinkacrylat, Zinkdiacrylat, Zinkmethacrylat, Zinkdimethacrylat, 1,2-Polybutadien oder N,N'-m-phenylen-dimaleinimid geeignet.

Die Gesamtmenge des oder der Vernetzer liegt üblicherweise im Bereich von 1 bis 20 phr, bevorzugt im Bereich von 1,5 bis 15 phr und besonders bevorzugt im Bereich von 2 bis 10 phr, bezogen auf den optional hydrierten Nitrilkautschuk.

Als Vernetzer können auch Schwefel in elementarer löslicher oder unlöslicher Form oder Schwefelspender eingesetzt werden.

Als Schwefelspender kommen beispielsweise Dimorpholyldisulfid (DTDM), 2-Morpholinodithiobenzothiazol (MBSS), Caprolactamdisulfid, Dipentamethylenthiuramtetrasulfid (DPTT), und Tetramethylthiuramdisulfid (TMTD) in Frage.

Auch bei der Schwefelvulkanisation der erfindungsgemäßen Nitrilkautschuke ist es möglich, noch weitere Zusätze zu verwenden, mit deren Hilfe die Vernetzungsausbeute erhöht werden kann. Grundsätzlich kann die Vernetzung aber auch mit Schwefel oder Schwefelspendern allein erfolgen. Umgekehrt kann die Vernetzung des erfindungsgemäßen, optional hydrierten Nitrilkautschuks aber auch nur in Gegenwart der oben genannten Zusätze erfolgen, d.h. ohne Zusatz von elementarem Schwefel oder Schwefelspendern.

Als Zusätze, mit deren Hilfe die Vernetzungsausbeute erhöht werden kann, eignen sich z.B. Dithiocarbamate, Thiurame, Thiazole, Sulfenamide, Xanthogenate, Guanidinderivate, Caprolactame und Thioharnstoffderivate.

Als Dithiocarbamate können beispielsweise eingesetzt werden: Ammoniumdimethyldithiocarbamat, Natriumdiethyldithiocarbamat (SDEC), Natriumdibutyl-dithiocarbamat (SDBC), Zinkdimethyldithiocarbamat (ZDMC), Zinkdiethyldithiocarbamat (ZDEC), Zinkdibutyldithiocarbamat (ZDBC), Zinkethylphenyldithiocarbamat (ZEPC), Zinkdibenzyldithiocarbamat (ZBEC), Zinkpentamethylendithiocarbamat (Z5MC), Tellurdiethyldithio-carbamat, Nickeldibutyldithiocarbamat, Nickeldimethyldithiocarbamat und Zinkdüsononyldithio-carbamat.

Als Thiurame können zum Beispiel eingesetzt werden: Tetramethylthiuramdisulfid (TMTD), Tetramethylthiurammonosulfid (TMTM), Dimethyldiphenylthiuramdisulfid, Tetrabenzylthiuramdisulfid, Dipentamethylenthiuramtetrasulfid und Tetraethylthiuramdisulfid (TETD).

Als Thiazole können zum Beispiel eingesetzt werden: 2-Mercaptobenzothiazol (MBT), Dibenzthiazyldisulfid (MBTS), Zinkmercaptobenzothiazol (ZMBT) und Kupfer-2-mercaptobenzo-thiazol. Als Sulfenamidderivate können zum Beispiel eingesetzt werden: N-Cyclohexyl-2-benzothiazylsulfenamid (CBS), N-tert.-Butyl-2-benzthiazylsulfenamid (TBBS), N,N'-Dicyclohexyl-2-benzthiazylsulfenamid (DCBS), 2-Morpholinothiobenzthiazol (MBS), N-Oxydiethylenthiocarbamyl-N-tert.butylsulfenamid und Oxydiethylenthiocarbamyl-N-oxyethylensulfenamid.

Als Xanthogenate können zum Beispiel eingesetzt werden: Natriumdibutylxanthogenat, Zinkisopropyldibutylxanthogenat und Zinkdibutylxanthogenat.

Als Guanidinderivate können zum Beispiel eingesetzt werden: Diphenylguanidin (DPG), Di-o-tolylguanidin (DOTG) und o-Tolylbiguanid (OTBG).
Als Dithiophosphate können beispielsweise eingesetzt werden: Zinkdialkydithiophosphate (Kettenlänge der Alkylreste C2 bis C16), Kupferdialkyldithiophosphate (Kettenlänge der Alkylreste C₂ bis C₁₆) und Dithiophoshorylpolysulfid.

Als Caprolactam kann beispielsweise Dithio-bis-caprolactam eingesetzt werden.

Als Thioharnstoffderivate können beispielsweise N,N'-Diphenylthioharnstoff (DPTU), Diethylthioharnstoff (DETU) und Ethylenthioharnstoff (ETU) eingesetzt werden.

Ebenso als Zusätze geeignet sind beispielsweise: Zinkdiamindiisocyanat, Hexamethylentetramin, 1,3-Bis(citraconimidomethyl)benzol sowie zyklische Disulfane.

Die genannten Zusätze als auch die Vernetzungsmittel können sowohl einzeln als auch in Mischungen eingesetzt werden. Bevorzugt werden folgende Substanzen für die Vernetzung der Nitrilkautschuke eingesetzt: Schwefel, 2-Mercaptobenzthiazol, Tetramethylthiuramdisulfid, Tetramethylthiurammonosulfid, Zinkdibenzyldithiocarbamat, Dipentamethylenthiuramtetrasulfid, Zinkdialkydithiophosphat, Dimorpholyldisulfid, Tellurdiethyldithiocarbamat, Nickeldibutyldithiocarbamat, Zinkdibutyldithiocarbamat, Zinkdimethyldithiocarbamat und Dithiobiscaprolactam.

Die Vernetzungsmittel und zuvor genannten Zusätze können jeweils in Mengen von ca. 0,05 bis 10 phr, vorzugsweise 0,1 bis 8 phr, insbesondere 0,5 bis 5 phr (Einzeldosierung, jeweils bezogen auf die Wirksubstanz) bezogen auf den optional hydrierten Nitrilkautschuk eingesetzt werden.

Bei der erfindungsgemäßen Schwefelvernetzung ist es gegebenenfalls auch sinnvoll, zusätzlich zu den Vernetzungsmitteln und oben genannten Zusätzen auch weitere anorganische bzw. organische Substanzen mit zu verwenden, beispielsweise: Zinkoxid, Zinkcarbonat, Bleioxid, Magnesiumoxid, Calciumoxid, gesättigte oder ungesättigte organische Fettsäuren und deren Zinksalze, Polyalkohole, Aminoalkohole, wie zum Beispiel Triethanolamin sowie Amine wie zum Beispiel Dibutylamin, Dicyclohexylamin, Cyclohexylethylamin und Polyetheramine.

Sofern es sich bei den erfindungsgemäßen gekuppelten, optional hydrierten Nitrilkautschuken um solche handelt, die Wiederholungseinheiten eines oder mehrerer carboxygruppenhaltiger Termonomere beinhalten, so kann ein Vernetzung auch über den Einsatz eines Polyamin-Vernetzers erfolgen, bevorzugt in Gegenwart eines Vernetzungsbeschleunigers. Der Polyamin-Vernetzer ist nicht eingeschränkt, solange es sich (1) um eine Verbindung handelt, die entweder zwei oder mehr Amino-Gruppen enthält (ggf. auch in Salz-Form) oder (2) um eine Spezies, die während der Vernetzungsreaktion in-situ eine Verbindung ausbildet, die zwei oder mehr Amino-Gruppen ausbildet. Bevorzugt wird eine aliphatische oder aromatische Kohlenwasserstoffverbindung eingesetzt bei der mindestens zwei Wasserstoffatome entweder durch Aminogruppen ersetzt sind oder aber durch Hydrazid-Strukturen (letzteres eine Struktur ,,-C(=O)NHNH₂").

Beispiele für derartige Polyamin-Vernetzer (ii) sind:
● Aliphatische Polyamine, bevorzugt Hexamethylendiamin, Hexamethylendiamincarbamat, Tetramethylenpentamin, Hexamethylendiaminzimtaldehyd-Addukt oder Hexamethylendiamindibenzoat;
● Aromatische Polyamine, bevorzugt 2,2-Bis(4-(4-aminophenoxy)phenyl) propan, 4,4'-Methylendianilin, m-Phenylendiamin, p-Phenylendiamin oder 4,4'-Methylen-bis(o-chloroanilin;
● Verbindungen mit mindestens zwei Hydrazid-Strukturen, bevorzugt Isophthalsäure dihydrazid, Adipinsäuredihydrazid oder Sebacinsäuredihydrazid.

Besonders bevorzugt sind Hexamethylendiamin und Hexamethylendiamincarbamat.

Die Menge des Polyamin-Vernetzeres in der vulkanisierbaren Mischung liegt üblicher Weise im Bereich von 0,2 bis 20 Gew.-Teile, bevorzugt im Bereich von 1 bis 15 Gew.-Teile und besonders bevorzugt im Bereich von 1,5 bis 10 Gew.-Teile bezogen auf 100 Gew.-Teile des optional hydrierten Nitrilkautschuks.

Als Vernetzungsbeschleuniger kann in Kombination mit dem Polyamin-Vernetzer jeder dem Fachmann bekannte eingesetzt werden, bevorzugt ein basischer Vernetzungsbeschleuniger. Einsetzbar sind z.B. Tetramethylguanidin, Tetraethylguanidin, Diphenylguanidin, Di-o-tolylguanidin (DOTG), o-Tolylbiguanidin und Di-o-tolylguanidin-Salz der Dicathecolborsäure. Einsetzbar sind ferner Aldehydeamin-Vernetzungsbeschleuniger wie z.B. n-Butylaldehydanilin. Besonders bevorzugt wird als Vernetzungsbeschleuniger mindestens eine bi- oder polycyclische aminische Base. Diese sind dem Fachmann bekannt. Insbesondere geeignet sind 1,8-diazabicyclo[5.4.0]undec-7-en (DBU), 1,5-diazabicyclo[4.3.0]-5-nonen (DBN), 1,4-diazabicyclo[2.2.2]octan (DABCO), 1,5,7-triazabicyclo[4.4.0]dec-5-en (TBD), 7-methyl-1,5,7-triazabicyclo[4.4.0]dec-5-en (MTBD).

Die Menge des Vernetzungsbeschleunigers liegt in diesem Fall üblicher Weise in einem Bereich von 0,5 bis 10 Gew.-Teile, bevorzugt 1 bis 7,5 Gew.-Teile, insbesondere 2 bis 5 Gew.-Teile, bezogen auf 100 Gew.-Teile des optional hydrierten Nitrilkautschuks.

Die vulkanisierbare Mischung basierend auf dem erfindungsgemäßen optional hydrierten Nitrilkautschuk kann prinzipiell auch Anvulkanisationsverzögerer enthalten. Hierzu gehören Cyclohexylthiophthalimid (CTP), N,N' Dinitrosopentamethlyentetramin (DNPT), Phthalsäureanhydrid (PTA) und Diphenylnitrosamin. Bevorzugt ist Cyclohexylthiophthalimid (CTP).

Neben der Zugabe des oder der Vernetzer kann der erfindungsgemäße, optional hydrierte Nitrilkautschuk auch mit weiteren üblichen Kautschukadditiven gemischt werden.

Als Füllstoffe können z.B. Ruß, Kieselsäure, Bariumsulfat, Titandioxid, Zinkoxid, Calciumoxid, Calciumcarbonat, Magnesiumoxid, Aluminiumoxid, Eisenoxid, Aluminiumhydroxid, Magnesiumhydroxid, Aluminiumsilikate, Diatomeenerde, Talkum, Kaoline, Bentonite, KohlenstoffNanotubes, Teflon (letzteres bevorzugt in Pulverform) oder Silikate eingesetzt werden.

Als Füllstoffaktivatoren kommen insbesondere organische Silane, wie beispielsweise Vinyltrimethyloxysilan, Vinyldimethoxymethylsilan, Vinyltriethoxysilan, Vinyltris(2-methoxy-ethoxy)silan, N-Cyclohexyl-3-aminopropyltrimethoxysilan, 3-Aminopropyl-trimethoxysilan, Methyltrimethoxysilan, Methyltlriethoxysilan, Dimethyldimethoxysilan, Dimethyldiethoxysilan, Trimethylethoxysilan, Isooctyltrimethoxysilan, Isooctyltriethoxysilan, Hexadecyltrimethoxysilan oder (Octadecyl)methyldimethoxysilan in Betracht. Weitere Füllstoffaktivatoren stellen zum Beispiel grenzflächenaktive Substanzen wie Triethanolamin und Ethylenglycole mit Molekulargewichten von 74 bis 10 000 g/mol dar. Die Menge an Füllstoffaktivatoren beträgt üblicherweise 0 bis 10 phr, bezogen auf 100 phr des optional hydrierten Nitrilkautschuks.

Als Alterungsschutzmittel können den vulkanisierbaren Mischungen aus der Literatur bekannte Alterungsschutzmittel zugesetzt werden. Sie werden üblicherweise in Mengen von ca. 0 bis 5 phr, bevorzugt 0,5 bis 3 phr, bezogen auf 100 phr des optional hydrierten Nitrilkautschuks eingesetzt.

Geeignete phenolische Alterungsschutzmittel sind alkylierte Phenole, styrolisiertes Phenol, sterisch gehinderte Phenole wie 2,6-Di-tert.-Butylphenol, 2,6-Di-*tert*-Butyl-*p*-Kresol (BHT), 2,6-Di-tert.-Butyl-4-Ethylphenol, estergruppenhaltige sterisch gehinderte Phenole, thioetherhaltige sterisch gehinderte Phenole, 2,2'-Methylen-bis-(4-Methyl-6-*tert*-Butylphenol) (BPH) sowie sterisch gehinderte Thiobisphenole.

Falls eine Verfärbung des Nitrilkautschuks ohne Bedeutung ist, werden auch aminische Alterungsschutzmittel z. B. Mischungen aus Diaryl-p-phenylendiaminen (DTPD), octyliertes Diphenylamin (ODPA), Phenyl-α-Naphthylamin (PAN), Phenyl-í-Naphthylamin (PBN), vorzugsweise solche auf Phenylendiaminbasis eingesetzt. Beispiele für Phenylendiamine sind *N-*Isopropyl-*N*'-phenyl-*p*-Phenylendiamin, *N*-1,3-Dimethylbutyl-*N*'-Phenyl-*p*-Phenylendiamin (6PPD), *N*-1,4-Dimethylpentyl-*N*'-phenyl-*p*-Phenylendiamin (7PPD) und *N,N*'-bis-1,4-(1,4-Dimethylpentyl)-*p*-Phenylendiamin (77PD).

Zu den sonstigen Alterungsschutzmitteln gehören Phosphite wie Tris-(nonylphenyl)phosphit, polymerisiertes 2,2,4-Trimethyl-1,2-dihydrochinolin (TMQ), 2-Mercaptobenzimidazol (MBI), Methyl-2-Mercaptobenzimidazol (MMBI), Zinkmethylmercaptobenzimidazol (ZMMBI). Die Phosphite werden im Allgemeinen in Kombination mit phenolischen Altrungsschutzmitteln eingesetzt. TMQ, MBI und MMBI werden vor allem dann verwendet, wenn peroxidisch vulkanisiert wird.

Als Formtrennmittel kommen beispielsweise in Betracht: Gesättigte und teilweise ungesättigte Fett- und Ölsäuren und deren Derivate (Fettsäureester, Fettsäuresalze, Fettalkohole, Fettsäureamide), die vorzugsweise als Mischungsbestandteil Verwendung finden, weiterhin auf die Formoberfläche applizierbare Produkte, wie beispielsweise Produkte auf Basis von niedermolekularen Silikonverbindungen, Produkte auf Basis von Fluorpolymeren sowie Produkte auf Basis von Phenolharzen.

Die Formtrennmittel werden als Mischungsbestandteil in Mengen von ca. 0 bis 10 phr, bevorzugt 0,5 bis 5 phr, bezogen auf 100 phr des optional hydrierten Nitrilkautschuks eingesetzt.

Auch die Verstärkung mit Festigkeitsträgern (Fasern) aus Glas, nach der Lehre von US-A-4,826,721 ist möglich sowie die Verstärkung durch Corde, Gewebe, Fasern aus aliphatischen und aromatischen Polyamiden (Nylon®, Aramid@), Polyestern und Naturfaserprodukten.

Gegenstand der vorliegenden Erfindung ist ferner ein Verfahren zur Herstellung von Vulkanisaten, dadurch gekennzeichnet, dass die zuvor genannte vulkanisierbare Mischung einer Vernetzung unterzogen wird. Die Vernetzung wird typischerweise entweder durch mindestens einen Vernetzer oder aber durch photochemische Aktivierung herbeigeführt.

Im Fall der photochemisch aktivierten Vulkanisation können als UV-Aktivatoren die dem Fachmann üblicherweise bekannten eingesetzt werden, beispielsweise Benzophenon, 2-Methylbenzophenon, 3,4-Dimethylbenzophenon, 3-Methylbenzophenon, 4,4'-Bis(diethylamino)benzophenon, 4,4'-Di-hydroxybenzophenon, 4,4'-Bis[2-(1-propenyl)phenoxy]benzophenon, 4-(Diethylamino) benzophenon, 4-(Dimethylamino)benzophenon, 4-Benzoylbiphenyl, 4-Hydroxybenzo-phenon, 4-Methylbenzophenon, Benzophenon-3,3',4,4'-tetracarboxyldianhydrid, 4,4'-Bis(dimethylamino) benzophenon, Acetophenon, 1-Hydroxycyclohexylphenylketon, 2,2-Diethoxyacetophenon, 2,2-Dimethoxy-2-phenylacetophenon, 2-Benzyl-2-(dimethylamino)-4'-morpholino-butyrophenon, 2-Hydroxy-2-methylpropiophenon, 2-Hydroxy-4'-(2-hydroxyethoxy)-2-methylpropiophenon, 3'-Hydroxyacetophenon, 4'-Ethoxyacetophenon, 4'-Hydroxyacetophenon, 4'-Phenoxyacetophenon, 4'-*tert*-Butyl-2',6'-dimethylacetophenon, 2-Methyl-4'-(methylthio)-2-morpholinopropiophenon, Diphenyl-(2,4,6-trimethylbenzoyl)-phosphinoxid, Phenylbis(2,4,6-trimethylbenzoyl)phosphinoxid, Methylbenzoylformat, Benzoin, 4,4'-Dimethoxybenzoin, Benzoinmethylether, Benzoinethylether, Benzoinisopropylether, Benzoinisobutylether, 4,4'-Dimethylbenzil, Hexachlorocyclopentadiene oder Kombinationen daraus.

### Beispiele:

Im Rahmen der folgenden Beispiele konnte mittels Massenspektrometrie (MS) eindeutig festgestellt werden, dass der erfindungsgemäße Nitrilkautschuk als Endgruppe der Polymerketten ein Regler-Fragment enthält, welches auf dem eingesetzten Regler basiert.

Folgende Abkürzungen werden im Folgenden an einigen Stellen verwendet:
- ACN: Acrylnitril
- 1,3-BD: 1,3-Butadien
- DoPAT: Dodecylpropansäuretrithiocarbonat
- Tetrazol-DoPAT: Tetrazol-funktionalisiertes Dodecylpropansäuretrithiocarbonat
- Vazo^{®} 88:: 1,1'-Azobis(cyclohexancarbonitril) (DuPont)
- M_{w}: Gewichtsmittel des Molekulargewichts
- Mₙ: Zahlenmittel des Molekulargewichts
- PDI: Polydispersitätsindex (Quotient aus M_{w} und Mₙ)
- DMF: Dimethylformamid

### Molekulargewichte sowie Polydispersitätsindex:

Die Bestimmung der Molekulargewichte in Form des Zahlenmittels des Molekulargewichts (Mₙ) und des Gewichtsmittels des Molekulargewichts (M_{w}) sowie des Polydispersitätsindex erfolgte mittels Gelpermeations-Chromatographie (GPC) nach DIN 55672-1 (Teil 1: Tetrahydrofuran THF als Lösungsmittel).

### Beispiel 1:

### Synthese des Tetrazol-funktionalen Trithiocarbonat-RAFT-Reglers 3-((2-(((dodecylthio)-carbonothioyl)thio)propanoyl)oxy)propyl 4-(2-phenyl-2H-tetrazol-5-yl)benzoate (Tetrazol-RAFT .

2-((Dodecylsulfanyl)carbonothioyl)-sulfanyl-propanolsäure (DoPAT, 5.000 g, 14.3 mmol), 1,3-Propandiol (5.2 mL, 71.3 mmol) und 4-(Dimethylamino)pyridin (0.346 g, 2.8 mmol) wurden in Tetrahydrofuran (10 mL) gelöst und mit einem Wasser/Eis-Bad auf 0 °C gekühlt. *N,N'-*Dicyclohexylcarbodiimide (2.940 g, 2.8 mmol) wurde zugegeben, das Kühlbad entfernt und die Mischung über Nacht bei Raumtemperatur gerührt. Der weiße Niederschlag wurde abfiltriert und verworfen. Das Filtrat wurde im Vakuum eingeengt und anschließend in Diethylether (200 mL) gelöst. Die Lösung wurde mit 1 M wässriger Salzsäurelösung (4 × 200 mL) extrahiert und anschließend mit gesättigter NaHCO₃-Lösung (200 mL) gewaschen. Die organische Phase wurde über MgSO₄ getrocknet und im Vakuum aufkonzentriert. Nebenprodukte wurden mittels Säulenchromatographie über Kieselgel mit Hexan/Essigester (3:1, v/v) abgetrennt, das Zwischenprodukt 3-hydroxypropyl 2-(((dodecylthio)carbonothioyl)thio)propanoate (Hydroxy-RAFT) mit Hexan/Essigester (1:1 v/v, R_{f} 0.61) von der Säule gewaschen und im Hochvakuum getrocknet. Ausbeute: 4.367 g, 74%, gelbes Öl.

Hydroxy-RAFT (3.800 g, 9.3 mmol), 4-(Dimethylamino)pyridin (0.026g, 0.02 mmol) und 4-(2-Phenyl-2H-Tetrazol-5-yl)benzoesäure (2.890 g, 10.9 mmol) wurden in 20 mL THF gelöst. Die Lösung wurde mit einem Wasser/Eis-Bad auf 0 °C gekühlt und *N,N'*-Dicyclohexylcarbodümide (2.450 g, 10.9 mmol) wurde zugegeben. Das Kühlbad wurde entfernt und die Reaktion über Nacht bei Raumtemperatur gerührt. Flüchtige Komponenten wurden anschließend im Vakuum entfernt und der Rückstand in Diethylether (200 mL) aufgenommen. Die organische Phase wurde mit 1 M wässriger Salzsäurelösung (4 × 200 mL) und anschließend mit gesättigter NaHCO₃ Lösung (200 mL) gewaschen, über MgSO₄ getrocknet und im Vakuum eingeengt. Das Rohprodukt wurde chromatographisch auf Kieselgel mit einem Laufmittelgemisch aus Hexan/Essigester (3:1, v/v, R_{f} 0.47) aufgereinigt und getrocknet. Tetrazol-RAFT wurde als gelber Feststoff erhalten. Ausbeute: 2.760 g, 45%.

### Beispiel 2:

### Herstellung Tetrazol-funktionaler NBR in organischer Lösung

Die Herstellung der in den folgenden Beispielserien eingesetzten Nitrilkautschuke erfolgte nach der für NBR 1 angegebenen Basisrezeptur unter den in Tabelle 1 angegebenen Bedingungen, wobei sämtliche Einsatzstoffe in Gew.-Teilen bezogen auf 100 Gew.-Teile der Monomermischung angegeben sind.

Sämtliche Apparaturen wurden vor Kontakt mit 1,3-Butadien durch dreimaliges Evakuieren und Spülen mit Stickstoff sauerstofffrei gemacht. In einer typischen Polymerisation wurden 44.3 mg Vazo 88 (0.13 phm) und 953.0 mg Tetrazol-RAFT (2.7 phm) in 18.8 mL Monochlorbenzol (59.1 phm) gelöst, 16.4 mL Acrylnitril (37.6 phm) zugesetzt und für 10 Minuten mit Stickstoff entgast. Die Monomer- / Initiatorlsg. wurde in den Reaktor überführt, dieser wurde verschlossen und durch dreimaliges Evakuieren / Spülen mit Stickstoff sauerstofffrei gemacht. 33.8 mL 1,3-Butadien (62.4 phm) wurden über eine Druckbürette zudosiert und die Reaktion durch Erhitzen auf 100 °C gestartet. Der Polymerisationsverlauf wurde durch gravimetrische Umsatzbestimmungen verfolgt. Nach 5 h wurde die Heizquelle entfernt, überschüssiges 1,3-Butadien nach Abkühlen des Reaktors durch Belüften entfernt und das Polymer durch Fällung in Ethanol erhalten. Das Polymer wurde anschließend im Hochvakuum getrocknet.

**Tabelle 1: Herstellung Tetrazol-funktionaler NBRs**

| **Bezeichnung:** | | **NBR 1** | **NBR 2** | **NBR 3** | **NBR 4** | **NBR 5** | **NBR 6** |
|---|---|---|---|---|---|---|---|
| **Rezeptur:** | | | | | | | |
| **Acrylnitril** | **phm** | 37.6 | 37.4 | 37.4 | 37.4 | 37.6 | 37.6 |
| **Butadien** | **phm** | 62.4 | 62.6 | 62.6 | 626 | 62.4 | 62.4 |
| **Monochlorbenzol** | **phm** | 59.1 | 58.9 | 59.2 | 59.2 | 59.1 | - |
| **Aceton** | **phm** | - | - | - | - | - | 42.4 |
| **Vazo^{®} 88** | **phm** | 0.13 | 0.084 | 0.017 | 0.0084 | 0.050 | 0.27 |
| **Tetrazol-RAFT** | **phm** | 2.7 | 3.6 | 0.44 | 0.11 | 1.1 | 10.7 |
| **Reaktionsbedingungen:** | | | | | | | |
| **Polymerisations-temperatur** | **°C** | 100 | 100 | 100 | 100 | 100 | 100 |
| **Polymerisationszeit** | **h** | 5 | 5.5 | 8 | 22 | 7 | 3 |
| **Endumsatz** | **%** | 18.0 | 18.5 | 10.8 | 8.9 | 12.0 | 37.2 |
| **Analytik:** | | | | | | | |
| **Mₙ** | **kg/mol** | 6.2 | 3.6 | 18 | 75 | 9.3 | 1.2 |
| **M_{W}** | **kg/mol** | 8.3 | 4.4 | 27 | 121 | 13 | 1.4 |
| **PDI** | | 1.3 | 1.2 | 1.5 | 1.6 | 1.4 | 1.2 |

### Beispiel 3:

### Kupplung Tetrazol-funktionaler NBRs

Die Kupplung der Nitrilkautschuke erfolgte nach der angegebenen Basisrezeptur unter den in Tabelle 2 angegebenen Bedingungen.

In einer typischen Kupplungsreaktion wurde Tetrazol-funktionaler NBR im angegebenen Lösemittel gelöst und die entsprechende Menge einer Stammlösung von 1,6-Bis(maleimido)hexan zugegeben. Die Reaktionsmischungen wurden 2-3 Stunden bei Raumtemperatur an Luft unter Rühren mit UV-Licht der Wellenlänge 254 nm bestrahlt. Die gekuppelten Polymere wurden durch Abtrennen des Lösemittels im Vakuum erhalten.

**Tabelle 2: Kupplung Tetrazol-funktionaler NBRs**

| **Bezeichnung:** | | **NBR 7** | **NBR 8** | **NBR 9** | **NBR 10** | **NBR 11** | **NBR 12** |
|---|---|---|---|---|---|---|---|
| **Rezeptur:** | | | | | | | |
| **Tetrazol-NBR** | **Nr.** | **NBR1** | **NBR2** | **NBR3** | **NBR4** | **NBR1** | **NBR1** |
| | **mg** | 30.0 | 40.0 | 40.0 | 120 | 30 | 30 |
| **1,6-Bis(maleimido)hexan** | **mg** | 1.02 | 2.10 | 0.44 | 0.33 | 1.02 | 1.02 |
| **Acetonitril** | **mL** | 50 | 6 | 6 | 6 | - | - |
| **Methylenchlorid** | **mL** | - | - | - | - | - | 50 |
| **Chlorbenzol** | **mL** | - | - | - | - | 50 | - |

| **Reaktionsbedingungen:** | | | | | | | |
|---|---|---|---|---|---|---|---|
| **Reaktionstemperatur** | **°C** | 25 | 25 | 25 | 25 | 25 | 25 |
| **Reaktionsszeit** | **min** | 120 | 180 | 180 | 180 | 120 | 120 |

| **Analytik:** | | | | | | | |
|---|---|---|---|---|---|---|---|
| **Mₙ** | **kg/mol** | 8.9 | 6.5 | 35 | 94 | 8.4 | 9.2 |
| **M_{w}** | **kg/mol** | 14 | 8.5 | 51 | 150 | 13 | 16 |
| **PDI** | | 1.6 | 1.3 | 1.5 | 1.6 | 1.5 | 1.7 |

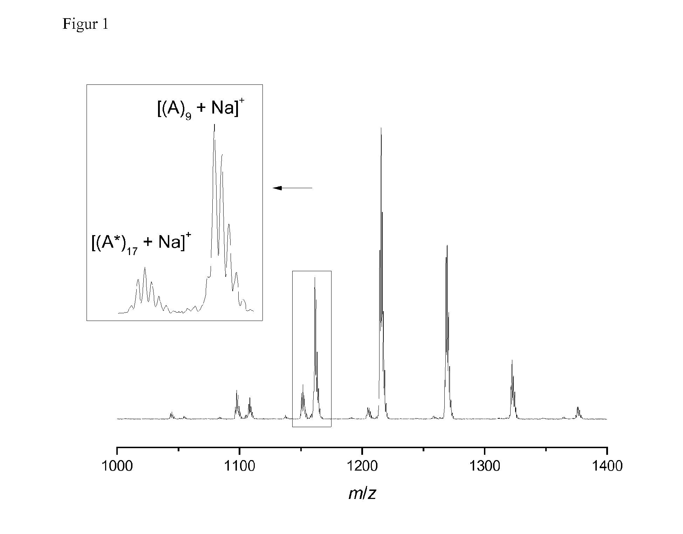

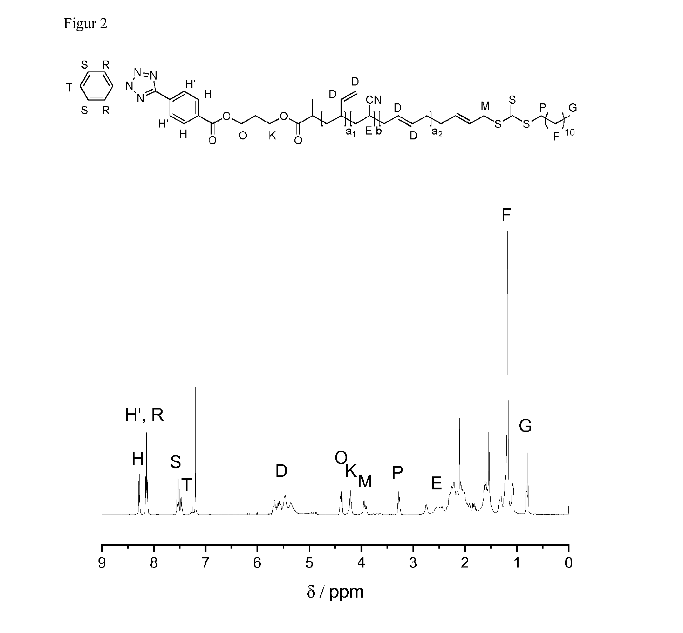

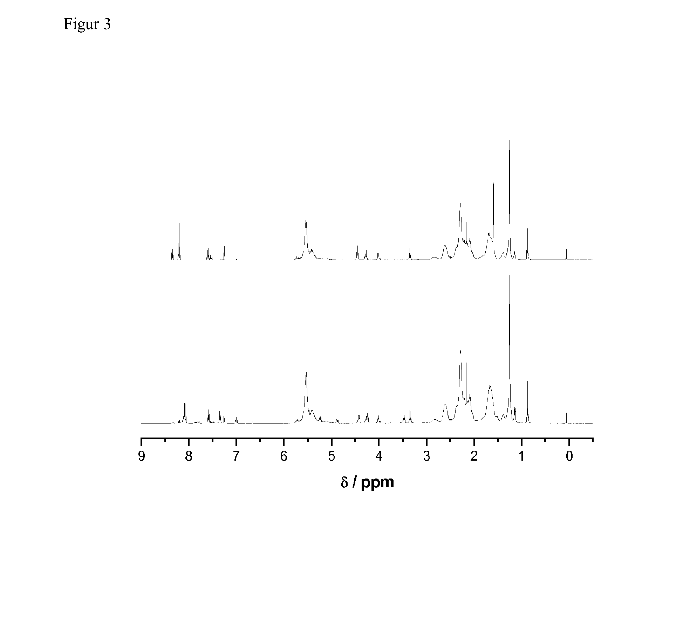

In den beigefügten Figuren 1 bis 3 sind der spektrometrische und der spektroskopische Nachweis der gebildeten Strukturen wiedergegeben.
**Fig. 1** zeigt einen Ausschnitt aus der SEC-massenspektrometrischen Analyse eines Tetrazol-funktionalisierten Nitrilkautschuks (NBR 6). Neben den Natrium-Addukten des Tetrazol-funktionalisierten Nitrilkautschuks A (in der Vergrößerung Nitrilkautschuk mit 9 Wiederholungseinheiten) werden außerdem kleine Mengen an Nitrilkautschuk A* (in der Vergrößerung Nitrilkautschuk mit 17 Wiederholungseinheiten) beobachtet, welche durch Rekombination zweier Ketten entstehen, deren Kettenenden mit Initiatorfragmenten besetzt sind.
**Fig. 2** zeigt eine H NMR-spektroskopische Analyse eines Tetrazol-funktionalisierten Nitrilkautschuks (NBR 6) und Zuordnung aller charakteristischen Resonanzen.
**Fig. 3** zeigt einen H NMR-spektroskopischen Vergleich eines Tetrazol-funktionalisierten Nitrilkautschuks (NBR 2, oben) mit dem durch UV-Bestrahlung daraus erhaltenen gekoppelten Nitrilkautschuk (NBR 8, unten).

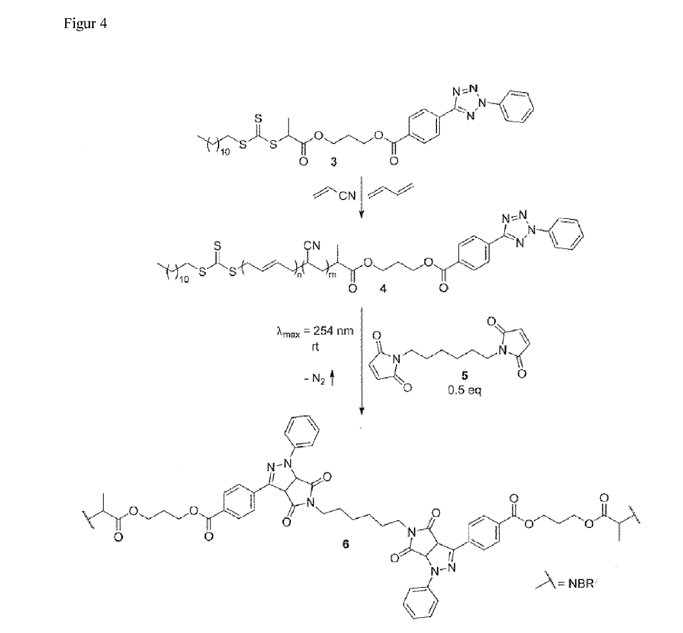

**Fig. 4** zeigt die Synthesestrategie des Beispiels und insbesondere die UV-induzierte molekulare Kopplung der NBR-Bausteine zur Bildung eines Nitrilkautschuks mit höherem Molekulargewicht. Die im Schema unten rechts dargestellte Struktur soll die NBR-Ketten wiedergeben.

## Patentansprüche

1. Verfahren zur Herstellung eines über Bisdihydropyrazol-Gruppen gekuppelten Nitrilkautschuks durch Umsetzung eines Nitrilkautschuks, der auf konjugierten Dienen, α,β-ungesättigten Nitrilen und gegebenenfalls weiteren copolymerisierbaren Monomeren als Monomeren basiert, hydriert sein kann und kovalent gebundene Tetrazolgruppen aufweist, mit einer bifunktionalen En-Verbindung, in der die En-Gruppen mit den Tetrazolgruppen jeweils zu Dihydropyrazol-Gruppen umgesetzt werden.

2. Verfahren nach Anspruch 1, **dadurch gekennzeichnet, dass** die Tetrazolgruppe als Rest der
allgemeinen Formel (1) 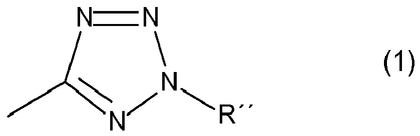 vorliegt, worin R" einen, gegebenenfalls substituierten, Arylrest darstellt.

3. Verfahren nach Anspruch 2, **dadurch gekennzeichnet, dass** der zu kuppelnde Nitrilkautschuk erhältlich ist durch radikalische Polymerisation mindestens eines konjugierten Diens, mindestens eines α,β-ungesättigten Nitrils und gegebenenfalls eines oder mehrerer weiterer copolymerisierbarer Monomere, in Gegenwart mindestens eines organischen Lösungsmittels und mindestens eines Reglers, wobei als Regler mindestens eine Verbindung der allgemeinen Formel (I) 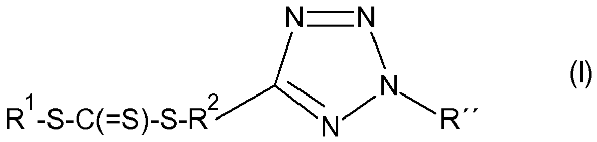 mit der Bedeutung
R¹ Kohlenwasserstoffrest, der substituiert sein kann und nach homolytischer Spaltung der R¹-S-Bindung ein primäres Radikal ausbildet,
R² Kohlenwasserstoffrest, der substituiert sein kann, eine oder mehrere Carboxylgruppen enthalten kann und nach homolytischer Spaltung der S-R²-Bindung ein sekundäres, tertiäres oder aromatisch stabilisiertes Radikal ausbildet,
R" wie in Anspruch 2 angegeben,
eingesetzt wird.

4. Verfahren nach Anspruch 3, **dadurch gekennzeichnet, dass** in der Verbindung der allgemeinen Formel (I) R¹ein C₈-C₁₅-Alkylrest ist und R² ein Rest -CHR³-C(=O)OR⁴- mit R³ C₁-C₃-Alkyl und R⁴ C₁-C₆-Alkylen, das durch Carboxyl- und/oder Arylgruppen unterbrochen sein kann, und R" Phenyl, Tolyl, Naphthyl oder Anthracenyl ist.

5. Verfahren nach einem der Ansprüche 1 bis 4, **dadurch gekennzeichnet, dass** in der bifunktionalen En-Verbindung zwei En-Gruppen der allgemeinen Formel (II)
R'-X-CH=CH-X-R' (II)
vorliegen, in der X jeweils unabhängig einen Rest C=O, CHOH, CHI, CHBr, CHCl, CHNO₂, CHNH₂ CHCOOH, CHC₆H₅, CHCN bedeutet und R' jeweils unabhängig einen C₁-C₁₂-Kohlenwasserstoffrest bedeutet, der ein oder mehrere Heteroatome enthalten kann, wobei die beiden Reste R' an einer En-Gruppe der allgemeinen Formel (II) zu einem Ring verbunden sein können und die beiden En-Gruppen der allgemeinen Formel (II) über jeweils mindestens einen der Reste R' kovalent miteinander verbunden sind.

6. Verfahren nach Anspruch 5, **dadurch gekennzeichnet, dass** die bifunktionale En-Verbindung zwei Maleinimidgruppen aufweist, deren Stickstoffatome über einen C₁-C₁₂-Alkylrest, der substituiert und von Heteroatomen und/oder Arylgruppen unterbrochen sein kann, miteinander verbunden sind.

7. Verfahren nach Anspruch 1 oder 2, **dadurch gekennzeichnet, dass** der zu kuppelnde Nitrilkautschuk erhältlich ist durch
a) radikalische Polymerisation mindestens eines konjugierten Diens, mindestens eines α,β-ungesättigten Nitrils und gegebenenfalls eines oder mehrerer weiterer copolymerisierbarer Monomere in Gegenwart mindestens eines Reglers und
b) optional anschließend eine Hydrierung,
wobei als Regler in Schritt a) mindestens eine Verbindung der allgemeinen Strukturformel (VI) eingesetzt wird, 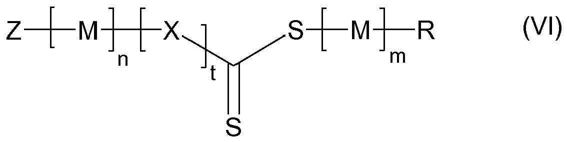 worin
Z für H, einen linearen oder verzweigten, gesättigten, ein- oder mehrfach ungesättigten Alkylrest, einen gesättigten, ein- oder mehrfach ungesättigten Carbo- oder Heterocyclylrest, Aryl, Heteroaryl, Arylalkyl, Heteroarylalkyl, Alkoxy, Aryloxy, Heteroaryloxy, Amino, Amido, Hydroxyimino, Carbamoyl, Alkoxycarbonyl, F, Cl, Br, I, Hydroxy, Phosphonato, Phosphinato, Alkylthio, Arylthio, Sulfanyl, Thiocarboxy, Sulfinyl, Sulfono, Sulfino, Sulfeno, Sulfonsäuren, Sulfamoyl, Silyl, Silyloxy, Nitril, Carbonyl, Carboxy, Oxycarbonyl, Oxysulfonyl, Oxo, Thioxo, Borate, Selenate, Epoxy, Cyanate, Thiocyanate, Isocyanate, Thioisocyanate und Isocyanide,
R **(a)** für den Fall, dass m ≠ 0 ist, die gleichen Bedeutungen besitzt wie der Rest Z und **(b)** für den Fall, dass m = 0 ist, für H, einen linearen oder verzweigten, gesättigten, ein-oder mehrfach ungesättigten Alkylrest, einen gesättigten, ein- oder mehrfach ungesättigten Carbo- oder Heterocyclylrest, Aryl, Heteroaryl, Arylalkyl, Heteroarylalkyl, Alkoxy, Aryloxy, Heteroaryloxy, Amino, Amido, Carbamoyl, Alkoxy, Aryloxy, Alkylthio, Arylthio, Sulfanyl, Thiocarboxy, Sulfinyl, Sulfono, Sulfino, Sulfeno, Sulfonsäuren, Sulfamoyl, Carbonyl, Carboxy, Oxycarbonyl, Oxysulfonyl, Oxo, Thioxo, Epoxy, Cyanate, Thiocyanate, Isocyanate, Thioisocyanate oder Isocyanide steht,
M für Wiederholungseinheiten eines oder mehrerer, ein- oder mehrfach ungesättigter Monomeren steht, umfassend konjugierte oder nicht-konjugierte Diene, Alkine und Vinylverbindungen, oder für ein Strukturelement, welches sich ableitet Polymeren umfassend Polyether, insbesondere Polyalkylenglykolether und Polyalkylenoxide, Polysiloxane, Polyole, Polycarbonate, Polyurethane, Polyisocyanate, Polysaccharide, Polyester und Polyamide,
n und m gleich oder verschieden sind und jeweils im Bereich von 0 bis 10.000 liegen,
t 0 oder 1 ist, sofern n= 0, und gleich 1 ist, sofern n ≠ 0, und
X für C(Z₂), N(Z), P(Z), P(=O)(Z), O, S, S(=O) oder S(=O)₂ steht, wobei Z in diesen Resten die gleichen Bedeutungen besitzt, wie zuvor für die Formel (VI) ausgeführt,
und nachfolgende Umsetzung des so erhaltenen Nitrilkautschuks mit einer Verbindung, die die kovalente Anbindung einer Tetrazolgruppe an den Nitrilkautschuk erlaubt und zu dieser Anbindung führt.

8. Über Bisdihydropyrazol-Gruppen gekuppelter Nitrilkautschuk.

9. Über Bisdihydropyrazol-Gruppen gekuppelter Nitrilkautschuk, erhältlich nach einem Verfahren gemäß einem der Ansprüche 1 bis 7.

10. Verwendung der über Bisdihydropyrazol-Gruppen gekuppelten Nitrilkautschuke nach Anspruch 8 oder 9 zur Herstellung von Formkörpern, Überzügen oder Vulkanisaten.

11. Vulkanisierbare Mischungen, enthaltend den Nitrilkautschuk nach einem der Ansprüche 8 oder 9, mindestens einen Vernetzer, optional mindestens einen Füllstoff und optional ein oder mehrere weitere Kautschukadditive.

12. Verfahren zur Herstellung von Vulkanisaten, **dadurch gekennzeichnet, dass** die vulkanisierbare Mischung nach Anspruch 11 einer Vernetzung unterzogen wird, bevorzugt durch Zusatz mindestens eines Vernetzers oder durch photochemische Aktivierung.

13. Vulkanisate, bevorzugt Formteile, erhältlich durch das Verfahren nach Anspruch 12.

14. Regler der allgemeinen Formel (I), wie er in einem der Ansprüche 3 oder 4 definiert ist.

15. Nitrilkautschuk, der kovalent gebundene Tetrazolgruppen aufweist und zur Kupplung über eine bifunktionale En-Verbindung geeignet ist, wie er in einem der Ansprüche 1 bis 4 definiert ist.
